# EUROPEAN PATENT SPECIFICATION

(11) **EP 4 688 126 B1**
(45) Date of publication and mention of the grant of the patent: **21.10.2026**
(21) Application number: 24711568.6
(22) Date of filing: 19.03.2024
(51) Int. Cl.: A61N 5/06, F21V 8/00

(54) **HUMAN-TISSUE TREATMENT DEVICE WITH LIGHT OUTPUT MEMBER USING TIR AT CONTACT SURFACE FOR IMPROVED SKIN- AND EYE-SAFETY**
VORRICHTUNG ZUR BEHANDLUNG VON MENSCHLICHEM GEWEBE MIT LICHTAUSGABEELEMENT UNTER VERWENDUNG TIR AN DER KONTAKTFLÄCHE FÜR VERBESSERTE HAUT- UND AUGENSICHERHEIT
DISPOSITIF DE TRAITEMENT DE TISSU HUMAIN AVEC ÉLÉMENT DE SORTIE DE LUMIÈRE UTILISANT DU TIR AU NIVEAU DE LA SURFACE DE CONTACT POUR AMÉLIORER LA SÉCURITÉ DE LA PEAU ET DES YEUX

(30) Priority: 29.03.2023 EP 23164906
(43) Date of publication of application: 11.02.2026
(73) Proprietor: Koninklijke Philips N.V., 5656 AE Eindhoven (NL)
(72) Inventor: PAULUSSEN, Elvira, Johanna, Maria, 5656 AG Eindhoven (NL); VARGHESE, Babu, 5656 AG Eindhoven (NL); BOAMFA, Marius, Iosif, 5656 AG Eindhoven (NL)
(74) Representative: Philips Intellectual Property & Standards
(86) International application number: PCT/EP2024/057210
(87) International publication number: WO 2024/200083

(56) References cited:
- US-A1- 2005 234 527
- US-A1- 2014 119 033
- US-A1- 2015 285 450

## Description

### FIELD OF THE INVENTION

The invention relates to a human-tissue treatment device comprising a housing, at least one light source arranged in the housing and configured to emit treatment light, a collimator configured and arranged to collimate the treatment light emitted by the light source and, thereby, to establish collimated treatment light having a first average propagation direction, and a plate-shaped light output member made from an optically transparent material and having a contact surface via which the collimated treatment light is transmissible to a human tissue when the human tissue is in contact with the contact surface.

The invention further relates to a shaving unit of an electric shaver, comprising a human-tissue treatment device as described hereinbefore.

The invention further relates to an electric shaver comprising a shaving unit as described hereinbefore.

### BACKGROUND OF THE INVENTION

A human-tissue treatment device as described hereinbefore in the section "field of the invention" is generally known. Among others, a system described in US2005/0234527 Al is suitable for skin treatments. The device may for example be used to treat human skin by light. The device may for example be used to treat acne vulgaris on the human skin by means of blue light or a combination of blue and red light. Another treatment example is hair removal or hair-growth reduction. By using collimated treatment light, the light intensity applied to the human tissue is relatively high, so that the treatment is effective. In particular, an effective treatment is achieved if the first average propagation direction of the collimated treatment light is transverse to, in particular perpendicular to the contact surface of the light output member. This however involves a safety risk in cases where the contact surface of the light output member is not covered or not fully covered by the human tissue. In such cases the collimated treatment light may reach the user's eyes, which may cause eye damage, or may reach parts of the human body that should not be treated.

To avoid such a safety risk, solutions are known to prevent the treatment light from being emitted via portions of the contact surface of the light output member which are not covered by the human tissue. In a known solution, a plurality of light sources is arranged along (parts of) the circumferential edges of the plate-shaped light output member, and the treatment light emitted by the light sources is optically coupled into the plate-shaped light output member via the circumferential edges thereof and guided through the plate-shaped light output member by total internal reflection (TIR) at the two main side surfaces of the plate-shaped light output member. Only in locations where the contact surface of the light output member is in contact with the human tissue, the treatment light will be coupled out of the light output member via the contact surface and into the human tissue. No treatment light will be emitted in locations where the contact surface is not in contact with the human tissue. A disadvantage of this solution is that the light intensity applied to the human tissue is limited due to the in-coupling of the treatment light via the relatively narrow circumferential edges of the plate-shaped light output member.

US 10,226,297 B2 discloses a medical instrument comprising mutually movable first and second jaw members cooperating to grasp tissue between the first and second jaw members. The first jaw member has a tissue-contacting surface and an optical element including a plurality of crystals made from an optically transparent material, such as sapphire, crystal glass or fused silica. One side of each crystal forms a portion of the tissue-contacting surface of the first jaw member. The first jaw member also includes multiple light sources that generate light beams which are directed into the respective crystals. Some of the light sources direct the light beams at a first angle into the crystals, such that the light beams are totally internally reflected and circulate within the crystals in a counter-clockwise direction when tissue is not in contact with the tissue-contacting surfaces of the crystals. The other light sources direct the light beams at a second angle different from the first angle into the crystals, such that the light beams are totally internally reflected and circulate within the crystals in a clockwise direction when tissue is not in contact with the tissue-contacting surfaces of the crystals. The light beams are transmitted into the tissue via only those crystals which are in contact with the tissue. Thus, no light is emitted from the crystals which are not in contact with the tissue, so that damage to the eyes by the light is prevented. A disadvantage of the known medical instrument is that the crystals which are not in contact with the tissue are heated by absorption of the light circulating in the crystals by total internal reflection. The heated crystals may damage any tissue with which they come into contact later.

### SUMMARY OF THE INVENTION

It is an object of the present invention to provide a human-tissue treatment device of a kind as described here before in the section "field of the invention", which does not have the disadvantages of the prior art devices as described here before in the section "background of the invention". In particular, it is an object of the present invention to provide a human-tissue treatment device of a kind as described in the section "field of the invention", wherein the collimated treatment light is applied from the collimator onto an inner main surface of the plate-shaped light output member opposite to the contact surface, so that a relatively high intensity of the treatment light applied to the human tissue is obtained, however without any safety risk of high-intensity treatment light being emitted from portions of the contact surface of the light output member which are not in contact with the human tissue, and without any risk of too much heating of the light output member by absorption of the collimated treatment light.

The invention is defined in the appended set of claims and explained in detail hereinafter. To achieve the above-mentioned objects, the present invention provides a human-tissue treatment device comprising a housing, at least one light source arranged in the housing and configured to emit treatment light, a collimator configured and arranged to collimate the treatment light emitted by the light source and, thereby, to establish collimated treatment light having a first average propagation direction, and a plate-shaped light output member made from an optically transparent material and having a contact surface via which the collimated treatment light is transmissible to a human tissue when the human tissue is in contact with the contact surface, wherein:
- an inner surface of the plate-shaped light output member opposite to the contact surface is provided with an optical structure having one or more pairs of a first facet and a second facet, the first facet and the second facet being oriented relative to the contact surface at, respectively, a first angle • 1 and a second angle • 2;
- the first facets are arranged to receive the collimated treatment light, the collimated treatment light having a second average propagation direction at a location where the collimated treatment light is incident on each respective first facet;
- the first angle • 1, the second average propagation direction and a refractive index value n1 of the optically transparent material of the plate-shaped light output member are such that: (i) the first facets transmit the received treatment light into the plate-shaped light output member by refraction; (ii) the contact surface reflects, at locations of no contact with the human tissue, the received treatment light by total internal reflection (TIR); and (iii) the contact surface transmits, at locations of contact with the human tissue, the received treatment light out of the plate-shaped light output member and into the human tissue by refraction;
- the second facets are arranged to receive the treatment light reflected by TIR at the contact surface; and
- the second angle • 2 and the refractive index value n1 are such that the second facets transmit the reflected treatment light out of the plate-shaped light output member by refraction.

In the human-tissue treatment device according to the invention, the treatment light collimated by the collimator is received by the first facets of the optical structure provided on the inner main surface of the plate-shaped light output member facing away from, i.e., arranged opposite to the contact surface of the plate-shaped light output member. Because the first facets are arranged on the inner main surface of the plate-shaped light output member and transmit the collimated treatment light by refraction into the plate-shaped light output member, i.e., without any substantial loss of light intensity, a relatively high amount of light can be received and transmitted into the plate-shaped light output member via the inner main surface of the plate-shaped light output member. When the contact surface of the plate-shaped light output member is in contact with the human tissue, for example human skin, the treatment light is transmitted into the human tissue by refraction at the contact surface, i.e., without any substantial loss of light intensity. At locations where the contact surface is not in contact with the human tissue, or in case of no contact at all of the contact surface with human tissue, the treatment light is totally internally reflected at the contact surface, so that the treatment light is not emitted to the environment of the device via the contact surface and may not cause any safety risks. The treatment light, which is totally internally reflected at the contact surface, is received by the second facets of the optical structure provided on the inner main surface of the plate-shaped light output member and is transmitted out of the plate-shaped light output member by refraction at the second facets. Thus, absorption of the treatment light by the plate-shaped light output member and the associated heating of the plate-shaped light output member are limited. The treatment light which is transmitted out of the plate-shaped light output member via the second facets may be absorbed by other parts of the human-tissue treatment device arranged in the housing thereof, so that heating of the plate-shaped light output member is limited.

In a preferred embodiment of the human-tissue treatment device according to the invention, the refractive index value n1 of the optically transparent material of the plate-shaped light output member is in a range from 1.4 to 1.7. A refractive index value n1 within the range from 1.4 to 1.7 enables an angle of incidence of the second average propagation direction of the collimated treatment light, relative to the contact surface of the plate-shaped light output member, in locations where the collimated treatment light is received by the first facets of the optical structure of the plate-shaped light output member to be relatively large, so that the light source can be arranged in a practical position within the housing. Suitable materials for the plate-shaped light output member which provide the refractive index value n1 within the range from 1.4 to 1.7 are, for example, polymethylmethacrylate (PMMA), polycarbonate, glass or quartz glass. Even higher values for said angle of incidence can be realized by using a material having a refractive index value n1 > 1.7, such as sapphire.

In a preferred embodiment of the human-tissue treatment device according to the invention, the first average propagation direction is different from the second average propagation direction, and the treatment device comprises a light-redirecting member arranged between the collimator and the plate-shaped light output member and configured to redirect the collimated treatment light from the first average propagation direction into the second average propagation direction. The use of the light-redirecting member allows the first average propagation direction of the treatment light collimated by the collimator to be different from the second average propagation direction of the collimated treatment light at the locations where the collimated treatment light is received by the first facets of the optical structure of the plate-shaped light output member. This allows the light source and the collimator to be positioned and oriented in a practical manner in the housing relative to the plate-shaped light output member. The light source and the collimator may, for example, be arranged centrally below the plate-shaped light output member in orientations such that an average light-emission direction of the light source and the first average propagation direction of the treatment light collimated by the collimator are perpendicular to the contact surface of the plate-shaped light output member.

In a further embodiment of the human-tissue treatment device according to the invention, the light-redirecting member comprises a plate-shaped carrier made from an optically transparent material and arranged parallel and adjacent to the inner surface of the plate-shaped light output member, wherein:
- a first main side of the plate-shaped carrier facing away from the plate-shaped light output member is flat, extends parallel to the contact surface, and is arranged to receive the collimated treatment light having the first average propagation direction and transmit the received collimated treatment light into the plate-shaped carrier;
- a second main side of the plate-shaped carrier facing the plate-shaped light output member is provided with an optical structure having one or more third facets oriented at a third angle •3 relative to the first main side and arranged to receive the collimated treatment light transmitted by the first main side; and
- the third angle •3, the first average propagation direction and a refractive index value n2 of the optically transparent material of the plate-shaped carrier are such that the third facets transmit and redirect the received collimated treatment light out of the plate-shaped carrier and into the second average propagation direction by refraction.

In this further embodiment, the plate-shaped carrier results in a practical and compact structure of the light-redirecting member and, together with the plate-shaped light output member arranged parallel and adjacent to the plate-shaped carrier, results in a practical and compact overall structure of the human-tissue treatment device. Because the collimated treatment light is received by the first main side of the plate-shaped carrier and is transmitted and redirected by refraction at the third facets of the optical structure provided at the second main side of the plate-shaped carrier, the light-redirecting member allows a high-intensity collimated treatment light beam to be transmitted and redirected from the first average propagation direction into the second average propagation direction without any substantial loss of light intensity.

In a yet further embodiment of the human-tissue treatment device in accordance with the invention, the optical structure of the second main side of the light-redirecting member comprises one or more pairs of a fourth facet and a respective one of the one or more third facets, the fourth facet being oriented relative to the first main side of the light-redirecting member at a fourth angle • 4, the third angle •3 being equal to the second angle • 2, and the fourth angle • 4 being equal to the first angle • 1. In this embodiment, the optical structure on the inner main surface of the plate-shaped light output member and the optical structure on the second main side of the plate-shaped carrier may be identical, so that in fact the plate-shaped light output member and the plate-shaped light-redirecting member may be identical, with the plate-shaped light-redirecting member being arranged in an inversed position relative to the plate-shaped light output member. This results in a simple and compact structure of the human-tissue treatment device.

In a preferred embodiment of the human-tissue treatment device in accordance with the invention:
- the collimator comprises an elongated light guide having first and second end surfaces and extending in a direction perpendicular to the contact surface from the first end surface to the second end surface;
- the first end surface is arranged to receive the treatment light emitted by the light source;
- the second end surface is arranged to transmit the collimated treatment light towards the light-redirecting member; and
- the light guide is configured to collimate the treatment light received at the first end surface by internal reflection of the received treatment light at a circumferential surface of the light guide.

This preferred embodiment is particularly suitable for devices having a single or a limited number of light sources arranged at a relatively large distance below the plate-shaped light output member and oriented such that an average light-emission direction of the light source(s) extends substantially perpendicularly to the contact surface of the plate-shaped light output member. In such devices, the light guide provides a practical and structurally simple embodiment of the collimator and provides a relatively high degree of collimation of the light emitted by the light source(s). The light guide may comprise a massive light-guiding body made from an optically transparent material and configured to collimate the treatment light received at the first end surface by TIR of the received treatment light at a circumferential surface of the light-guiding body. Alternatively, the light guide comprises a hollow light-guiding channel configured to collimate the treatment light received at the first end surface by reflection of the received treatment light at a reflective inner surface of the light-guiding channel.

In a further embodiment of the human-tissue treatment device in accordance with the invention:
- the light guide has a central axis extending perpendicularly to the contact surface and is circularly symmetric relative to the central axis; and
- the light guide has a cross-sectional area perpendicular to the central axis which gradually increases from the first end surface towards the second end surface.

In this further embodiment, when the light source is arranged centrally on the central axis of the light guide and has an average light-emission direction in the direction of the central axis, the light guide provides a uniform collimation of the light emitted by the light source, seen over the cross-section of the light guide, and the degree of collimation of the emitted light is relatively high. In this further embodiment, the light guide may be embodied as a compound parabolic concentrator (CPC). Preferably, in this further embodiment the first and second facets of the optical structure of the plate-shaped light output member are annular and extend concentrically relative to the central axis of the light guide, and, if the light-redirecting member comprises the plate-shaped carrier with the optical structure according to an embodiment as described here before, also the third and (when applicable) the fourth facets of the optical structure of the light-redirecting member are annular and extend concentrically relative to the central axis.

A particular embodiment of the human-tissue treatment device according to the invention comprises a plurality of light sources configured to emit the treatment light, wherein the collimator comprises a separate collimating element for each respective light source configured and arranged to collimate the treatment light emitted by the respective light source. In this embodiment, the light sources may be arranged at a relatively short distance below the plate-shaped light output member and, despite said short distance, may provide a uniform light intensity at the contact surface when regularly distributed over the entire area of the plate-shaped light output member. This embodiment is preferred when only a limited amount of space is available below the plate-shaped light output member for accommodating the light sources and the collimator. This embodiment may comprise a light-redirecting member as described here before, in which case each respective light source may have an average light-emission direction perpendicular to the contact surface of the plate-shaped light output member, and the first average propagation direction of the light collimated by the collimating element associated with each respective light source may be perpendicular to the contact surface as well. The light-redirecting member may have the plate-shaped carrier with the optical structure according to an embodiment as described here before. In an alternative embodiment, the light-redirecting member may comprise a separate light-redirecting element for each respective light source arranged between the plate-shaped light output member and the collimating element associated with the respective light source. In this alternative embodiment, said light-redirecting elements may each comprise a mirror or a prism.

In another embodiment of the human-tissue treatment device according to the invention, which comprises a plurality of light sources configured to emit the treatment light:
- the collimator comprises a separate collimating element for each respective light source configured and arranged to collimate the treatment light emitted by the respective light source; and
- each respective light source and each respective collimating element associated with the respective light source are oriented obliquely relative to the contact surface of the plate-shaped light output member, such that the first average propagation direction and the second average propagation direction are mutually parallel.

Because in this embodiment the first average propagation direction and the second average propagation direction are mutually parallel as a result of the oblique orientation of light sources and the collimating elements, a light-redirecting member or a separate light-redirecting element for each respective light source is not required. The light sources can be arranged at a relatively short distance below the plate-shaped light output member, so that only a limited space is required below the plate-shaped light output member for accommodating the light sources.

In a further embodiment of the human-tissue treatment device according to the invention:
- the collimator comprises a plurality of optical fibers arranged to guide the treatment light emitted by the light source to the plate-shaped light output member;
- the optical fibers have fiber tip portions which are oriented such that the treatment light emitted by the fiber tip portions has the second average propagation direction; and
- the optical fibers are configured to receive the treatment light emitted by the light source and maintain the received treatment light with a numerical aperture value in a range from 0.01-0.2.

In this further embodiment, as a result of the use of the optical fibers, the light source may be arranged in any suitable or desired position within the housing, which provides an increased flexibility in designing the overall layout of the human-tissue treatment device. Because in this further embodiment the fiber tip portions are oriented such that the treatment light emitted by the fiber tip portions has the second average propagation direction, a light-redirecting member is not required. The numerical aperture value of the treatment light in the range from 0.01-0.2, received and maintained by the optical fiber, ensures that the treatment light emitted by the fiber tip portions is sufficiently collimated.

In a still further embodiment of the human-tissue treatment device according to the invention, which comprises a light-redirecting member as described here before:
- the collimator comprises a plurality of optical fibers arranged to guide the treatment light emitted by the light source to the light-redirecting member;
- the optical fibers have fiber tip portions which are oriented perpendicularly to the contact surface of the plate-shaped light output member; and
- the optical fibers are configured to receive the treatment light emitted by the light source and maintain the received treatment light with a numerical aperture value in a range from 0.01-0.2.

In this still further embodiment, the orientation of the fiber tip portions perpendicular to the contact surface of the plate-shaped light output member results in a first average propagation direction of the collimated treatment light which is perpendicular to the contact surface.

The present invention further provides a shaving unit of an electric shaver, comprising a base member, at least one hair-cutting unit supported by the base member, and at least one human-tissue treatment device according to the invention as described hereinbefore, wherein the contact surface of the plate-shaped light output member of the human-tissue treatment device is arranged relative to the hair-cutting unit such that the contact surface and the hair-cutting unit are together in contact with skin of a user during use of the shaving unit. Thus, the human-tissue treatment device may provide an additional light treatment to the skin of the user during use of the shaving unit, i.e., with the hair-cutting unit and the contact surface of the human-tissue treatment device being in contact with the skin of the user, while any risk of eye damage by the collimated treatment light generated by the human-tissue treatment device is prevented during periods of no contact of the shaving unit with the skin.

A preferred embodiment of the shaving unit according to the invention further comprises a skin-supporting member having a skin-supporting surface which at least partially surrounds the hair-cutting unit, wherein the human-tissue treatment device is arranged in the skin-supporting member such that the contact surface of the plate-shaped light output member of the human-tissue treatment device forms a portion of the skin-contacting surface of the skin-supporting member. This preferred embodiment provides a practical arrangement of the human-tissue treatment device in the shaving unit, wherein the contact surface of the human-tissue treatment device may at least partially surround the hair-cutting unit.

The present invention further provides an electric shaver comprising a main body and a shaving unit according to the invention as described hereinbefore, wherein the main body accommodates an electric motor, and wherein the shaving unit is arranged on the main body such that the hair-cutting unit of the shaving unit is drivable by the electric motor.

The above-described and other aspects of the invention will be apparent from and elucidated with reference to the following detailed description of embodiments of a human-tissue treatment device in accordance with the invention.

### BRIEF DESCRIPTION OF THE DRAWINGS

The invention will be explained in greater detail with reference to the figures, in which equal or similar features are indicated by the same reference signs, and in which:
Fig. 1 schematically shows a top view of a first embodiment, a second embodiment and a third embodiment of a human-tissue treatment device according to the invention;
Fig. 2 schematically shows a cross-section of the first embodiment of the human-tissue treatment device along the line II-II in Fig. 1;
Fig. 3 schematically shows a cross-section of a collimating element of a collimator of the first, second and third embodiment of the human-tissue treatment device of Fig. 1;
Figs. 4a and 4b schematically show the propagation of treatment light in a light output member of the first and second embodiment of the human-tissue treatment device of Fig. 1 in conditions of, respectively, contact and no contact of the light output member with human tissue;
Fig. 5 schematically shows a cross-section of the second embodiment of the human-tissue treatment device along the line V-V in Fig. 1;
Fig. 6 schematically shows a light source of the second embodiment of the human-tissue treatment device of Fig. 1 together with an associated light-redirecting element;
Fig. 7 schematically shows a detail of the top view of the third embodiment of the human-tissue treatment device indicated by the reference VII in Fig. 1;
Fig. 8 schematically shows a cross-section of a light output member and a light-redirecting member of the third embodiment of the human-tissue treatment device along the line VIII-VIII in Fig. 7;
Fig. 9 schematically shows a cross-section of a fourth embodiment of a human-tissue treatment device according to the invention;
Fig. 10 schematically shows a top view of the human-tissue treatment device of Fig. 9;
Fig. 11 schematically shows a fifth embodiment of a human-tissue treatment device according to the invention;
Fig. 12 shows a shaving unit of an electric shaver according to the invention, provided with a human-tissue treatment device according to the invention;
Fig. 13 schematically shows a cross-section of a skin-supporting member of the shaving unit extending through the line XIII-XIII in Fig. 12; and
Fig. 14 schematically shows a top view of the human-tissue treatment device of the shaving unit of Fig. 12.

### DETAILED DESCRIPTION OF THE EMBODIMENTS

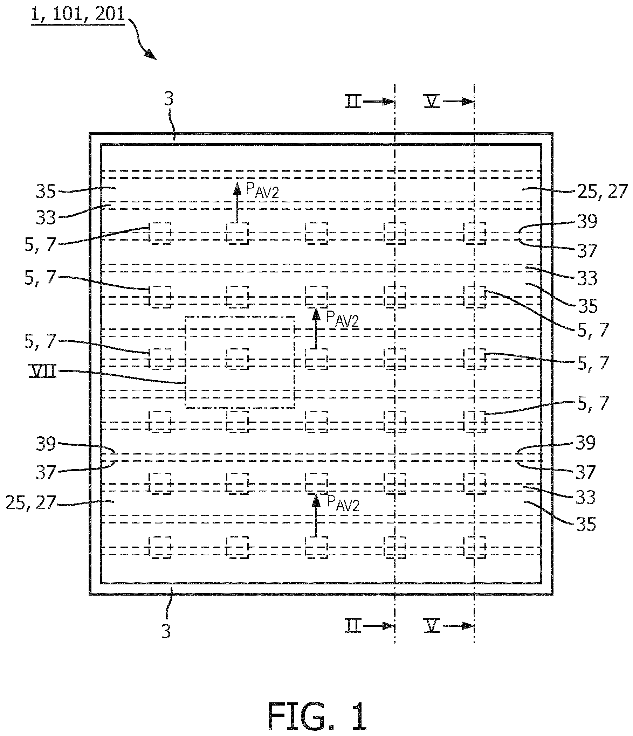

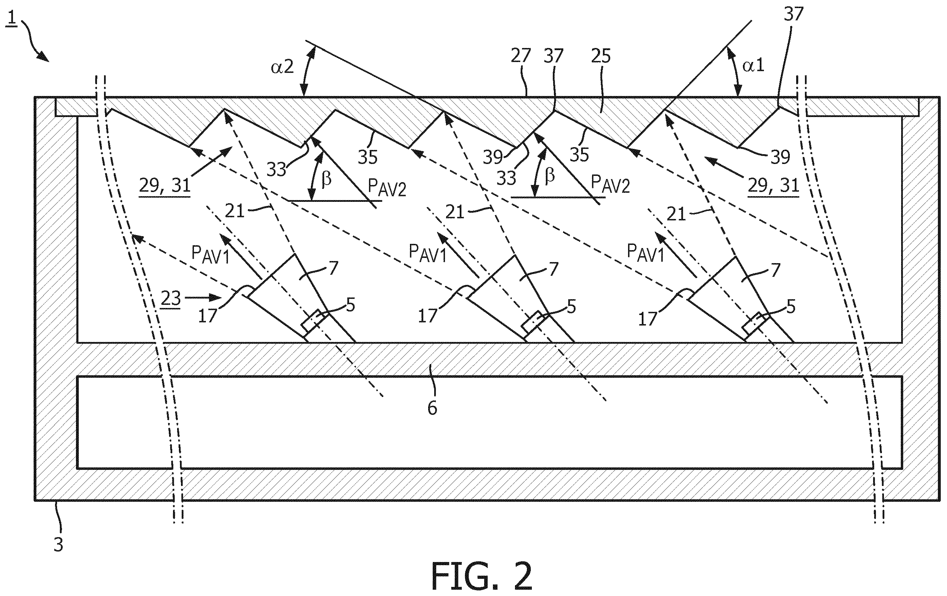

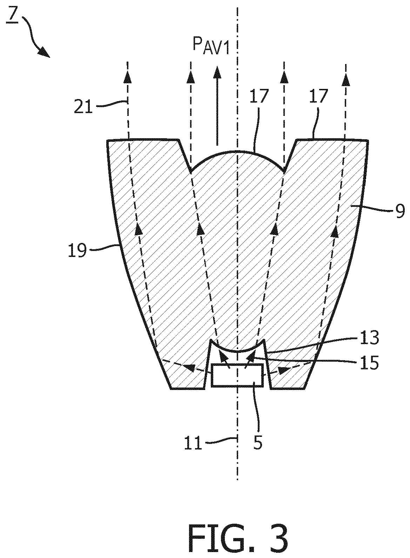

Fig. 1 and Fig. 2 schematically show a first embodiment of a human-tissue treatment device 1 according to the invention. The human-tissue treatment device 1 comprises a housing 3 wherein a plurality of light sources 5 are arranged. The light sources 5 are each supported by a supporting member 6, which is also arranged in the housing 3. The light sources 5 are each configured to emit treatment light and may each comprise, for example, an LED. In the first embodiment as shown in Fig. 2, a separate collimating element 7 is associated with each respective light source 5 and is configured and arranged to collimate the treatment light emitted by the respective light source 5. The collimating element 7 may, for example, be of a kind as schematically shown in Fig. 3, comprising a solid body 9 made from an optically transparent material and having a central axis 11. The solid body 9 is circularly symmetric relative to the central axis 11 and has a first end surface 13, arranged to receive the treatment light 15 emitted by the light source 5 associated with the collimating element 7, and a second end surface 17. The shapes of the first and second end surfaces 13, 17 and the shape of an outer circumferential surface 19 of the solid body 9 are such that collimated treatment light 21 having a first average propagation direction P_{AV1} is established at the second end surface 17 by refraction of the treatment light 15 at the first and second end surfaces 13, 17 and by total internal reflection (TIR) of the treatment light 15 at the outer circumferential surface 19. This way of light collimation is well known to the skilled person and will not be discussed in further detail. The collimating elements 7 together form a collimator 23 of the human-tissue treatment device 1, which is configured and arranged to collimate the treatment light 15 emitted by the light sources 5 and, thereby, to establish collimated treatment light 21 having the first average propagation direction P_{AV1}.

As shown in Fig. 1 and Fig. 2, the human-tissue treatment device 1 further comprises a plate-shaped light output member 25 made from an optically transparent material. An outer main surface of the plate-shaped light output member 25 constitutes a contact surface 27 of the plate-shaped light output member 25, which is arranged to contact human tissue during use of the human-tissue treatment device 1. An inner main surface 29 of the plate-shaped light output member 25 has an average extension parallel to the contact surface 27 and is arranged opposite to the contact surface 27 to receive the collimated treatment light 21. As will be explained in detail hereinafter, the collimated treatment light 21 is transmissible to the human tissue via the plate-shaped light output member 25 and the contact surface 27 thereof when the human tissue is in contact with the contact surface 27. The human tissue may for example be human skin, and the treatment light may for example comprise blue light or a combination of blue light and red light of a suitable intensity to treat acne vulgaris. Combinations of the light intensity and duration of the treatment light suitable for the treatment are well known to the skilled person and will not be discussed here. Other types of treatment of the human skin, such as hair removal or hair-growth reduction, or the treatment of other types of human tissue, such as gum tissue, may be possible as well by means of a human-tissue treatment device according to the invention.

As shown in Fig. 2, the inner main surface 29 of the plate-shaped light output member 25 is provided with an optical structure 31 which has a plurality of pairs of a first facet 33 and a second facet 35. Seen in the cross-section of Fig. 2, the first facets 33 are each oriented relative to the contact surface 27 at a first angle • 1 and the second facets 35 are each oriented relative to the contact surface 27 at a second angle • 2. In the embodiment shown, the respective first and second facets 33, 35 mutually connect at respective edges 37, 39 of the first and second facets 33, 35. The first facets 33 are arranged to receive the collimated treatment light 21 directly from the collimator 23, i.e. only via air present between the collimator 23 and the plate-shaped light output member 25. As a result, a second average propagation direction P_{AV2} of the collimated treatment light 21 at a location where the collimated treatment light 21 is incident on each respective first facet 33 is parallel to the first average propagation direction P_{AV1} of the collimated treatment light 21 emitted by the collimating elements 7. Because, as shown in Fig. 2, in this embodiment each respective light source 5 and the respective collimating element 7 associated with the respective light source 5 are oriented obliquely relative to the contact surface 27 of the plate-shaped light output member 25, the first and second average propagation directions P_{AV1} and P_{AV2} are inclined at an angle of incidence • relative to the contact surface 27. As shown in Fig. 1, in this embodiment the edges 37, 39 of the first and second facets 33, 35 extend rectilinearly and parallel to each other and, in a viewing direction perpendicular to the contact surface 27, the second average propagation direction P_{AV2} extends substantially perpendicularly to the edges 37, 39 of the first and second facets 33, 35.

According to the present invention, the first angle • 1 of the first facets 33, the second average propagation direction P_{AV2}, i.e., the angle of incidence • of the collimated treatment light 21 relative to the contact surface 27 at the first facets 33, and a refractive index value n1 of the optically transparent material of the plate-shaped light output member 25 are such that:
- (i) the first facets 33 transmit the collimated treatment light 21 received by the first facets 33 into the plate-shaped light output member 25 by refraction;
- (ii) the contact surface 27 of the plate-shaped light output member 25 reflects, at locations of no contact with the human tissue, the collimated treatment light received by the first facets 33 by total internal reflection (TIR); and
- (iii) the contact surface 27 of the plate-shaped light output member 25 transmits, at locations of contact with the human tissue, the collimated treatment light received by the first facets 33 out of the plate-shaped light output member 25 and into the human tissue by refraction.

Furthermore, according to the present invention, the second facets 35 are arranged to receive the collimated treatment light which is totally internally reflected at the contact surface 27 according to the above-mentioned optical effect (ii), and the second angle • 2 of the second facets 35 and the refractive index value n1 of the optically transparent material of the plate-shaped light output member 25 are such that:
- (iv) the second facets 35 transmit the collimated treatment light, which is totally internally reflected at the contact surface 27, out of the plate-shaped light output member 25 by refraction.

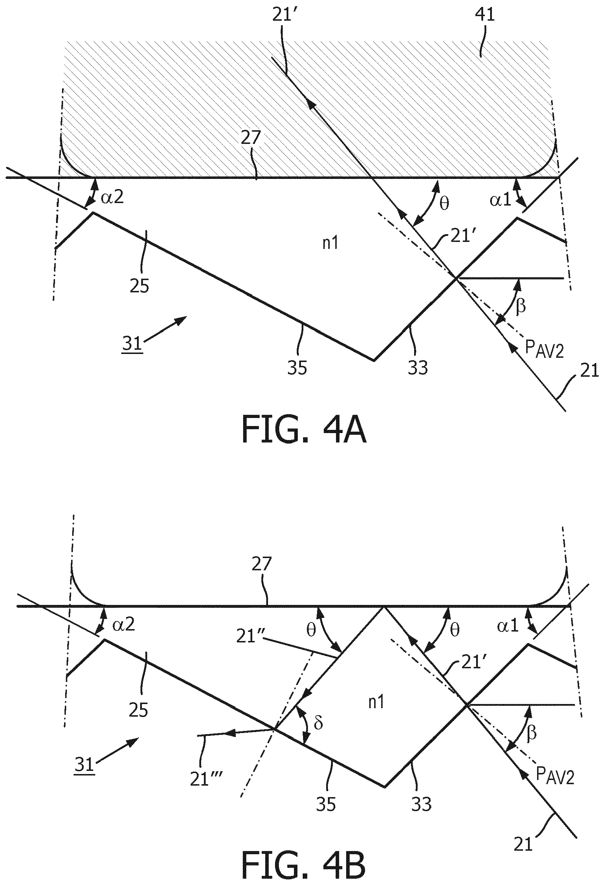

The combination of the above-mentioned optical effects (i) and (iii) is schematically illustrated in Fig. 4a for a single pair of a first facet 33 and a second facet 35 of the optical structure 31, with the contact surface 27 being in contact with human tissue 41. In Fig. 4a, the collimated treatment light 21 incident on the first facet 33 at the angle of incidence • relative to the contact surface 27 is refracted by the first facet 33 and, as a result, propagates as collimated treatment light 21' in the plate-shaped light output member 25. Because the contact surface 27 is in contact with the human tissue 41, the collimated treatment light 21' is transmitted out of the plate-shaped light output member 25 and into the human tissue 41 by refraction at the contact surface 27.

The combination of the above-mentioned optical effects (i), (ii) and (iv) is schematically illustrated in Fig. 4b for a single pair of a first facet 33 and a second facet 35 of the optical structure 31, with the contact surface 27 not being in contact with human tissue. In Fig. 4b, the collimated treatment light 21 incident on the first facet 33 at the angle of incidence • relative to the contact surface 27 is refracted by the first facet 33 and, as a result, propagates as collimated treatment light 21' in the plate-shaped light output member 25. Because there is no human tissue in contact with the contact surface 27, the collimated treatment light 21' is totally internally reflected at the contact surface 27 and, as a result, propagates further in the plate-shaped light output member 25 as collimated treatment light 21". The collimated treatment light 21" is received by the second facet 35, is transmitted out of the plate-shaped light output member 25 by refraction at the second facet 35 and propagates further into the air outside of the plate-shaped light output member 25 as collimated treatment light 21‴.

As a result of the TIR of the collimated treatment light 21' at the contact surface 27 in case of no contact of the contact surface 27 with the human tissue 41, as described hereinbefore, the treatment light emitted by the light sources 5 is prevented from being emitted via the contact surface 27 into the environment of the human-tissue treatment device 1 in case the contact surface 27 is not covered by the human tissue 41. When the contact surface 27 is partially covered by the human tissue 41, the treatment light is prevented from being emitted into the environment of the human-tissue treatment device 1 via the portions of the contact surface 27 which are not covered by the human tissue 41. As a result, the treatment light emitted by the light sources 5 is prevented from reaching, for example, the eyes of the user of the human-tissue treatment device 1 or parts of the human body, in particular skin tissue, that should not be treated. Furthermore, because the collimated treatment light 21", that is internally reflected at the contact surface 27, is transmitted out of the plate-shaped light output member 25 by refraction at the second facets 35, absorption of the treatment light by the plate-shaped light output member 25 and associated heating of the plate-shaped light output member 25 are limited. The collimated treatment light 21"', which is transmitted out of the plate-shaped light output member 25 via the second facets 35, may be absorbed by the housing 3 or by other parts of the human-tissue treatment device 1 (not shown in the figures) arranged in the housing 3, so that heating of the plate-shaped light output member 25 is limited.

Because the collimated treatment light 21 is received by the optical structure 31 arranged on the inner main surface 29 of the plate-shaped light output member 25 and transmitted into the plate-shaped light output member 25 by refraction at the first facets 33 of the optical structure 31, i.e., without any substantial loss of light intensity, a relatively high amount of collimated treatment light can be received by the inner main surface 29 of the plate-shaped light output member 25 and transmitted to the human tissue 41 via the contact surface 27. In the first embodiment, the light sources 5 may be arranged at a relatively short distance below the plate-shaped light output member 25, so that the necessary volume of the human-tissue treatment device 1 below the plate-shaped light output member 25 can be reduced. When the light sources 5 are regularly distributed over the entire area of the plate-shaped light output member 25, e.g., when the light sources 5 are distributed according to a regular 2D-array as shown in Fig. 1, a uniform light intensity at the contact surface 27 may be achieved despite the relatively short distance between the light sources 5 and the plate-shaped light output member 25. It is noted that the number of light sources 5 and the number of first and second facets 33, 35 of the optical structure 31 shown in Fig. 1 is purely illustrative. A skilled person will be able to determine in a straight-forward manner the number and distribution of the light sources 5 required to obtain a desired level of uniformity of the light intensity at the contact surface 27. Seen in the cross-section of Fig. 2, a single light source 5 may be associated with a single pair of a first and a second facet 33, 35. Alternatively, as shown in Fig. 2, a single light source 5 may be associated with two or more pairs of a first and a second facet 33, 35. In this context, the skilled person will also be able to determine in a straight-forward manner the number and the dimensions of the first and second facets 33, 35, in particular as seen in the cross-section of Fig. 2.

Although in the most preferred embodiments of the human-tissue treatment device according to the invention the optical structure 31 of the plate-shaped light output member 25 may have a plurality of pairs of a first facet 33 and a second facet 35, in some embodiments the optical structure may have only one pair of a first facet and a second facet, in particular in embodiments wherein the contact surface 27 of the plate-shaped light output member 25 has a relatively small area. It will be evident for the skilled person that, for a predefined area of the contact surface 27, the number of pairs of first and second facets 33, 35 can be decreased by increasing the dimensions of the first and second facets 33, 35. The increased dimensions of the first and second facets 33, 35 will however result in an increased height (thickness) of the optical structure 31 perpendicular to the contact surface 27 and, as a result, in an increased thickness of the plate-shaped light output member 25.

Furthermore, based on the explanation of the optical effects of the optical structure 31 as described in detail here before, the skilled person will be able to determine in a straight-forward manner any suitable values for the first angle • 1, the second angle • 2, the second average propagation direction P_{AV2} (i.e., the angle of incidence • ) of the collimated treatment light 21 relative to the contact surface 27 at the first facets 33, and the refractive index value n1 of the optically transparent material of the plate-shaped light output member 25, e.g., based on Snell's law of optical refraction and the critical angle of incidence for TIR which can be calculated using Snell's law.

The refractive index value n1 is preferably in a range from 1.4 to 1.7. For example, for n1 = 1.5 TIR at the contact surface 27 occurs if the angle of incidence • , relative to the contact surface 27, of the collimated treatment light 21' propagating in the plate-shaped light output member 25 (see Fig. 4a) is about 48° or smaller. If the first facets 33 are oriented perpendicularly to the second average propagation direction P_{AV2}, the first angle • 1 may be about 42° or larger, and the angle of incidence • of the collimated treatment light 21 relative to the contact surface 27 at the first facets 33 may be about 48° or smaller. Using also the effect of optical refraction of the collimated treatment light 21 at the first facets 33, the angle • 1 may be even smaller than about 42°, and the angle of incidence • may be even larger than 48°. In this case, the second average propagation direction P_{AV2} will generally be directed transversely but not perpendicularly to the first facets 33. This will result in a more favorite position of the light sources 5 relative to the plate-shaped light output member 25.

Based on Snell's law, it is evident for the skilled person that higher values of the refractive index value n1 will result in higher values for the angle of incidence • of the collimated treatment light 21 at the first facets 33 and lower values for the angle • 1 and will therefor result in an even more favorite position of the light sources 5 relative to the plate-shaped light output member 25. Suitable materials for the plate-shaped light output member 25, which provide the refractive index value n1 within the range from 1.4 to 1.7, are for example polymethylmethacrylate (PMMA), polycarbonate, glass or quartz glass. Even higher values for the angle of incidence • of the collimated treatment light 21 at the first facets 33 can be realized by using a material having a refractive index value n1 > 1.7, such as sapphire.

The angle • 2 of the second facets 35 may be determined in a straight-forward manner based on the requirement that the angle of incidence • , relative to the second facets 35, of the collimated treatment light 21" internally reflected at the contact surface 27 (see Fig. 4b) should be larger than the critical angle of incidence at which TIR occurs at the second facets 35. Based on Snell's law, the angle of incidence • should be larger than about 48° if n1 = 1.5. In practice this may result in a relatively small angle • 2, which will minimize the required thickness of the optical structure 31 seen perpendicularly to the contact surface 27.

Furthermore, the degree of collimation of the collimated treatment light 21 by the collimator 23 may be taken into account when determining the first angle • 1, the second angle • 2, the angle of incidence • of the collimated treatment light 21 at the first facets 33, and the refractive index value n1. In practice the rays of the collimated treatment light 21 will not be perfectly parallel, i.e., the collimated treatment light 21 will still be slightly divergent. Based on the degree of divergence of the collimated treatment light 21, the first angle • 1, the second angle • 2, the angle of incidence • of the collimated treatment light 21 at the first facets 33, and the refractive index value n1 may be determined such that the collimated treatment light 21' propagating in the plate-shaped light output member 25 is fully internally reflected at the contact surface 27 in case of no contact of the contact surface 27 with the human tissue 41, or such that at least a major portion of the collimated treatment light 21' is internally reflected at the contact surface 27 at such a degree that eye-safe conditions are obtained.

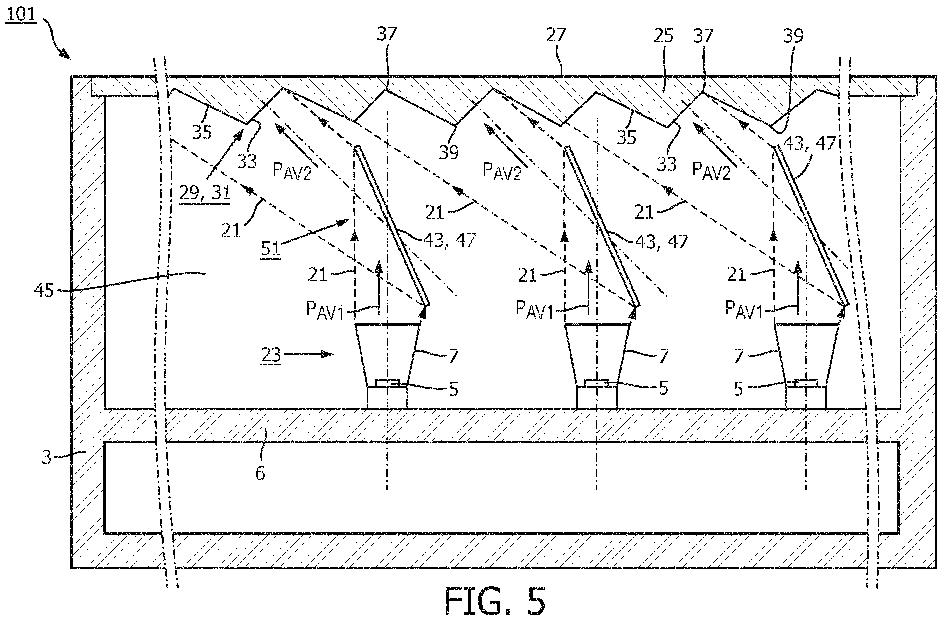

Fig. 1 and Fig. 5 schematically show a second embodiment of a human-tissue treatment device 101 according to the invention. In the cross-section of Fig. 5, features of the human-tissue treatment device 101, which are identical and corresponding to features of the first embodiment of the human-tissue treatment device 1 as discussed in detail hereinbefore, are indicated by corresponding reference numbers. Hereinafter, such identical and corresponding features are not discussed in detail, and only the main different features of the human-tissue treatment device 101 are discussed in detail.

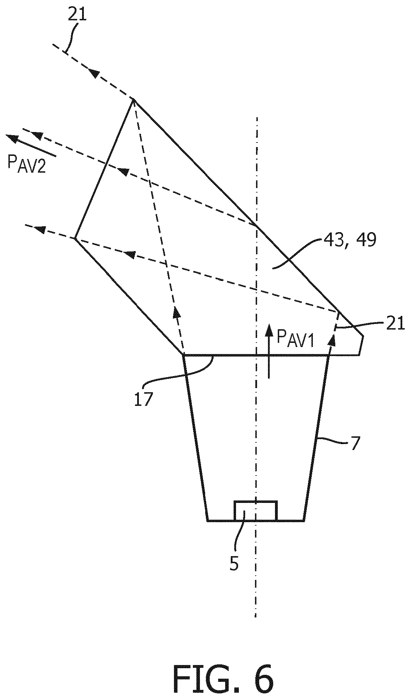

Similar to the first embodiment of the human-tissue treatment device 1, the human-tissue treatment device 101 comprises a plurality of light sources 5 (e.g., LEDs) arranged on a supporting member 6 in a housing 3 and configured to emit treatment light. Similar to the human-tissue treatment device 1, the human-tissue treatment device 101 comprises a collimator 23 which has a separate collimating element 7 for each respective light source 5 configured and arranged to collimate the treatment light emitted by the respective light source 5. The collimating elements 7 may each be of the type as shown in Fig. 3 and as discussed in detail hereinbefore. Whereas in the human-tissue treatment device 1 the second average propagation direction P_{AV2} of the collimated treatment light 21 at the first facets 33 of the optical structure 31 of the plate-shaped light output member 25 is parallel to the first average propagation direction P_{AV1} of the collimated treatment light 21 emitted by the collimating elements 7, in the human-tissue treatment device 101 the first average propagation direction P_{AV1} is different from the second average propagation direction P_{AV2}. In particular, as shown in Fig. 5, the light sources 5 and the associated collimating elements 7 are mounted to the supporting member 6 such that the first average propagation direction P_{AV1} is substantially perpendicular to the contact surface 27 of the plate-shaped light output member 25. To redirect the collimated treatment light 21 emitted by the collimating elements 7 from the first average propagation direction P_{AV1} into the second average propagation direction P_{AV2}, the human-tissue treatment device 101 comprises a separate light-redirecting element 43 for each respective light source 5, which is arranged in the air-filled space 45 between the collimating element 7, associated with the respective light source 5, and the plate-shaped light output member 25. In the second embodiment as shown in Fig. 5, each light-redirecting element 43 comprises a mirror 47, which is shown only schematically in Fig. 5 and may be mounted to the associated collimating element 7 or to a suitable support structure (not shown in Fig. 5) for all mirrors 47. Alternatively, each light-redirecting element 43 may comprise a prism 49. Fig. 6 schematically shows one of the light sources 5 of the human-tissue treatment device 101, together with the associated collimating element 7 and the associated light-redirecting prism 49.

In the second embodiment of the human-tissue treatment device 101, the light-redirecting elements 43 together form a light-redirecting member 51 of the human-tissue treatment device 101, which is arranged between the collimator 23 and the plate-shaped light output member 25 and configured to redirect the collimated treatment light 21 emitted by the collimator 23 from the first average propagation direction P_{AV1} into the second average propagation direction P_{AV2}. Generally, the use of the light-redirecting member 51 allows the first average propagation direction P_{AV1} of the collimated treatment light 21 emitted by the collimator 23 to be different from the second average propagation direction P_{AV2} of the collimated treatment light 21 at the locations where the collimated treatment light 21 is received by the first facets 33 of the optical structure 31 of the plate-shaped light output member 25. This allows the light sources 5 to be positioned in any desired practical orientation on the supporting member 6. For example, the supporting member 6 may be a printed circuit board (PCB), and the light sources 5 may be LEDs mounted in normal upright positions on the PCB.

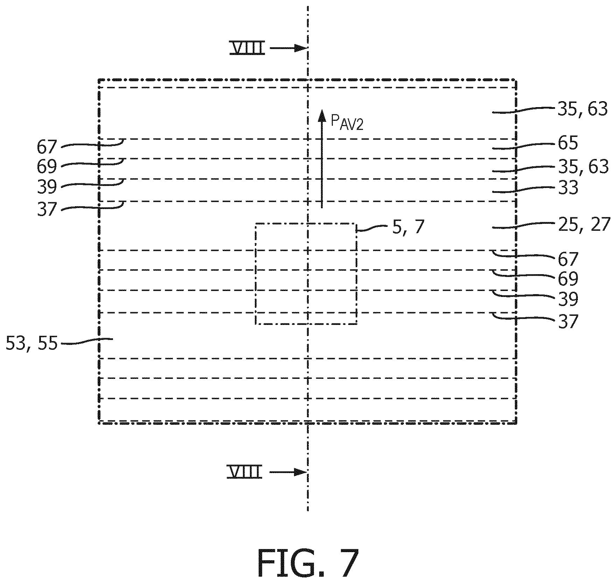

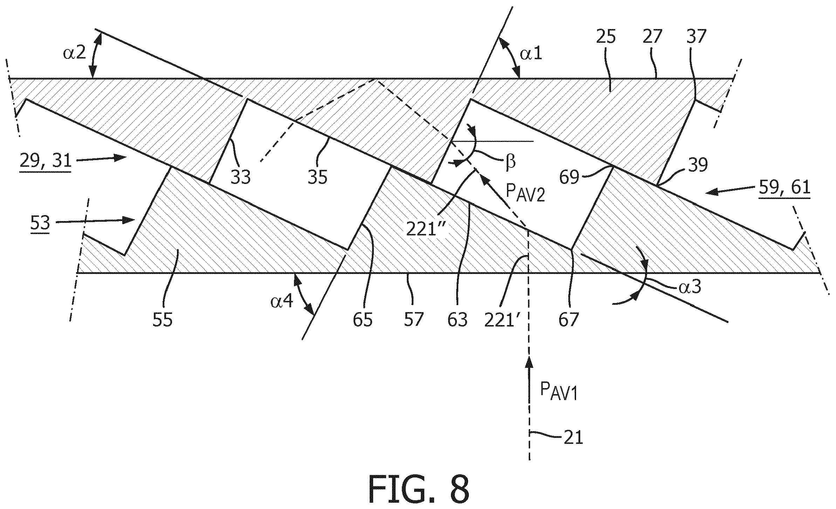

A third embodiment of the human-tissue treatment device 201 according to the invention, also schematically shown in Fig. 1, is similar to the second embodiment of the human-tissue treatment device 101 as described hereinbefore and has a plurality of light sources 5 and associated collimating elements 7, wherein each light source 5 (e.g., LED) and its associated collimating element 7 are arranged on the supporting member 6 (e.g., PCB) such that the first average propagation direction P_{AV1} of the collimated treatment light 21 emitted by the collimating elements 7 is substantially perpendicular to the contact surface 27 of the plate-shaped light output member 25. A main difference between the human-tissue treatment device 201 according to the third embodiment and the human-tissue treatment device 101 according to the second embodiment is that in the human-tissue treatment device 201 the light-redirecting elements 43, used in the human-tissue treatment device 101, are replaced by a single light-redirecting member 53 (see Fig. 8) which is common for all light sources 5. The light-redirecting member 53 of the human-tissue treatment device 201 comprises a plate-shaped carrier 55 made from an optically transparent material and arranged parallel and adjacent to the inner main surface 29 of the plate-shaped light output member 25. The plate-shaped carrier 55 of the light-redirecting member 53 is partially visible in Fig. 7, which only shows in a schematic way a detail of the top view of the human-tissue treatment device 201 indicated by the reference VII in Fig. 1, and in Fig. 8, which schematically shows a cross-section of the plate-shaped light output member 25 and the plate-shaped carrier 55 of the light-redirecting member 53 along the line VIII-VIII in Fig. 7. Although the plate-shaped carrier 55 is not fully visible in Fig. 7 and Fig. 8, it should be understood that the plate-shaped carrier 55 extends along the full area of the plate-shaped light output member 25.

As shown in Fig. 8, a first main side 57 of the plate-shaped carrier 55 of the light-redirecting member 53, facing away from the plate-shaped light output member 25, is flat and extends parallel to the contact surface 27 of the plate-shaped light output member 25. The first main side 57 of the plate-shaped carrier 55 is arranged to receive the collimated treatment light 21, emitted by the collimating elements 7 (not shown) and having the first average propagation direction P_{AV1}, and to transmit the collimated treatment light 21 into the plate-shaped carrier 55.

A second main side 59 of the plate-shaped carrier 55 of the light-redirecting member 53, facing the plate-shaped light output member 25, is provided with an optical structure 61 having a plurality of pairs of a third facet 63 and a fourth facet 65. Seen in the cross-section of Fig. 8, the third facets 63 are each oriented relative to the first main side 57 at a third angle • 3, and the fourth facets 65 are each oriented relative to the first main side 57 at a fourth angle • 4. In the embodiment shown, the respective third and fourth facets 63, 65 mutually connect at respective edges 67, 69 of the third and fourth facets 63, 65. As shown in Fig. 7, the edges 67, 69 of the third and fourth facets 63, 65 of the optical structure 61 of the light-redirecting member 53 extend rectilinearly, parallel to each other, and parallel to the edges 37, 39 of the first and second facets 33, 35 of the optical structure 31 of the plate-shaped light output member 25. Although the third and fourth facets 63, 65 are not fully visible in Fig. 7 and Fig. 8, it should be understood that the third and fourth facets 63, 65 extend along the full extension of the first and second facets 33, 35, and that each respective pair of a first facet 33 and a second facet 35 of the optical structure 31 of the plate-shaped light output member 25 is associated with a respective pair of a third facet 63 and a fourth facet 65 of the optical structure 61 of the light-redirecting member 53 in a similar manner as schematically shown in Fig. 7 and Fig. 8.

As shown in Fig. 8, the third facets 63 of the optical structure 61 of the light-redirecting member 53 are arranged to receive the collimated treatment light 221' which is transmitted by the first main side 57 of the plate-shaped carrier 55. In this embodiment, the third angle • 3, the first average propagation direction P_{AV1} of the collimated treatment light 21 incident on the first main side 57, and a refractive index value n2 of the optically transparent material of the plate-shaped carrier 55 are such that the third facets 63 transmit and redirect, by refraction, the collimated treatment light 221' out of the plate-shaped carrier 55 and into the second average propagation direction P_{AV2} towards the first facets 33 of the plate-shaped light output member 25. For a predefined value of the first average propagation direction P_{AV1} of the collimated treatment light 21 emitted by the collimating elements 7, the skilled person will be able to determine in a straight-forward manner, e.g., based on Snell's law of optical refraction, any suitable values for the third angle • 3 and the refractive index value n2 of the optically transparent material of the plate-shaped carrier 55 required to achieve any desired value of the second average propagation direction P_{AV2} of the collimated treatment light 221" at the first facets 33, i.e., the angle of incidence • of the collimated treatment light 21 as described in detail hereinbefore with respect to the first embodiment of the human-tissue treatment device 1. When the first average propagation direction P_{AV1} is perpendicular to the first main side 57 of the plate-shaped carrier 55, as in the third embodiment of the human-tissue treatment device 201, and the refractive index value n2 is for example 1.5, while the third angle • 3 is about 40°, the collimated treatment light 221" is transmitted by the third facets 63 out of the plate-shaped carrier 55 into a second average propagation direction P_{AV2} which has an angle of incidence • of about 55° relative to the contact surface 27. It is evident for the skilled person that the angle of incidence • can be decreased by increasing the refractive index value n2 and/or by increasing the third angle • 3.

The light-redirecting member 53 has a compact structure and, together with the plate-shaped light output member 25 arranged immediately adjacent and parallel to the light-redirecting member 53, results in a practical and compact overall structure of the human-tissue treatment device 201. Because the collimated treatment light 21 emitted by the collimating elements 7 is received by the first main side 57 of the plate-shaped carrier 55 and is transmitted and redirected by refraction at the third facets 63 provided at the second main side 59 of the plate-shaped carrier 55, the light-redirecting member 53 allows a high amount of collimated treatment light 21 to be transmitted and redirected from the first average propagation direction P_{AV1} into the second average propagation direction P_{AV2} without any substantial loss of light intensity.

Although in the third embodiment of the human-tissue treatment device 201 according to the invention the optical structure 61 of the light-redirecting member 53 has a plurality of pairs of a third facet 63 and a fourth facet 65, in some embodiments the optical structure of the light-redirecting member may have only one pair of a third facet and a fourth facet, in particular in embodiments wherein the contact surface 27 of the plate-shaped light output member 25 has a relatively small area. It will be evident for the skilled person that, for a predefined area of the contact surface 27, the number of pairs of third and fourth facets 63, 65 can be decreased by increasing the dimensions of the third and fourth facets 63, 65. The increased dimensions of the third and fourth facets 63, 65 will however result in an increased height (thickness) of the optical structure 61 perpendicular to the contact surface 27 and, as a result, in an increased thickness of the light-redirecting member 53.

Preferably, as in the third embodiment shown schematically in Fig. 8, the third angle • 3 of the third facets 63 of the light-redirecting member 53 is equal to the second angle • 2 of the second facets 35 of the plate-shaped light output member 25, and the fourth angle • 4 of the fourth facets 65 of the light-redirecting member 53 is equal to the first angle • 1 of the first facets 33 of the plate-shaped light output member 25. As a result, the optical structure 31 on the inner main surface 29 of the plate-shaped light output member 25 and the optical structure 61 on the second main side 59 of the light-redirecting member 53 may be identical, so that in fact the plate-shaped light output member 25 and the plate-shaped light-redirecting member 53 may be identical, with the plate-shaped light-redirecting member 53 being arranged in an inversed position relative to the plate-shaped light output member 25 as shown in Fig. 8. This results in a simple and compact structure of the human-tissue treatment device 201. The condition that the third angle • 3 equals the second angle • 2 and that the fourth angle • 4 equals the first angle • 1 may be obtained without affecting the desired optical effects of the plate-shaped light output member 25 and the light-redirecting member 53, because the fourth facets 65 do not have a dominant role in the optical function of the light-redirecting member 53 (i.e., the fourth angle • 4 is not crucial) and because the range of allowable values of the second angle • 2 is relatively wide in practice.

The human-tissue treatment devices 1, 101, 201 according to the first, second and third embodiments of the invention as described hereinbefore each comprise a plurality of light sources 5 which are arranged at a relatively short distance below the plate-shaped light output member 25 to provide a uniform light intensity at the contact surface 27. This results in a relatively compact structure of the human-tissue treatment devices 1, 101, 201. The invention also covers embodiments wherein, to provide a uniform light intensity at the contact surface of the plate-shaped light output member by means of only a single light source or a small number of light sources, the light source(s) is/are arranged at a relatively large distance below the plate-shaped light output member. In these embodiments, the dimensions of the human-tissue treatment device might not be a critical design factor, or the contact surface of the plate-shaped light output member may have a relatively small area, e.g., when the human-tissue treatment device is intended for the treatment of relatively small areas of the human tissue, such as for example individual pimples on the human skin.

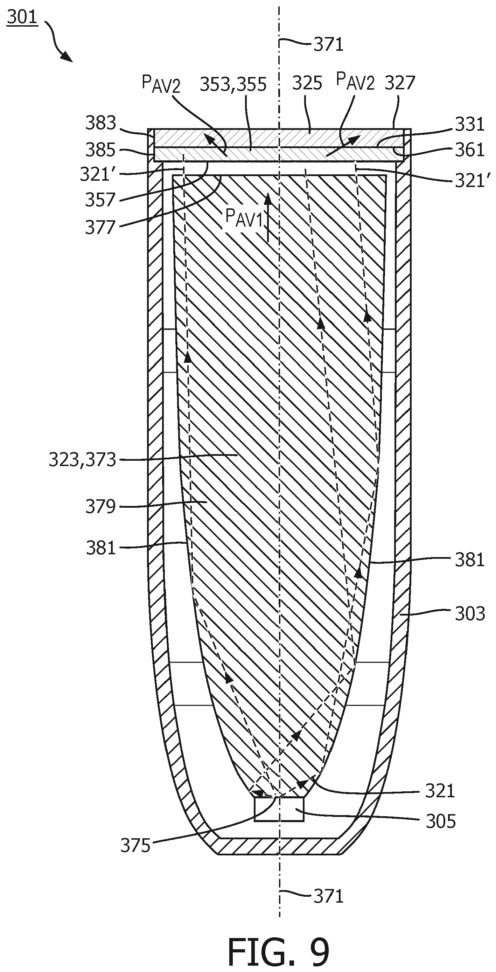

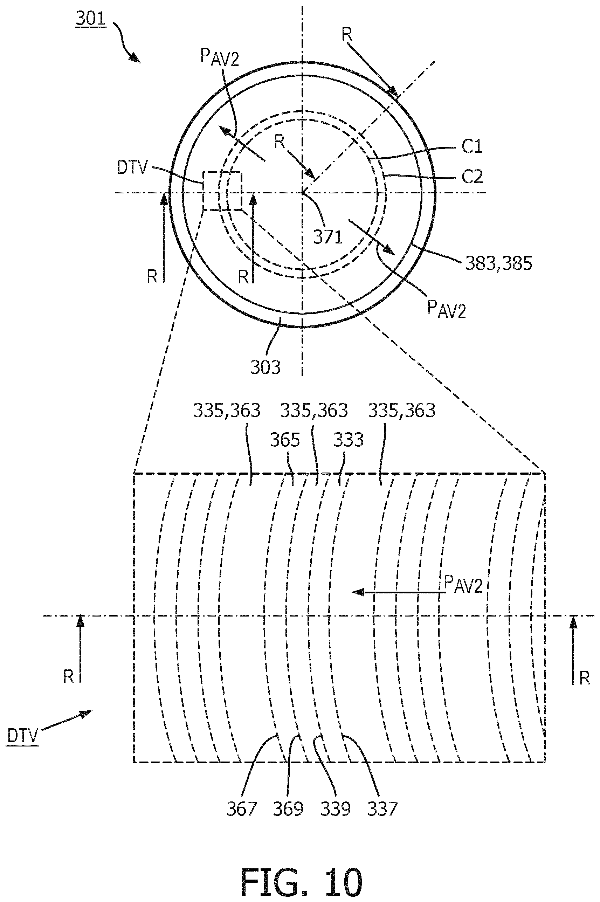

The fourth embodiment of a human-tissue treatment device 301 according to the invention, as schematically shown in Fig. 9 and Fig. 10, is an acne-treatment device having an elongated housing 303 and a contact surface 327 with a circular circumferential edge arranged at a top end of the housing 303. The contact surface 327 may have a relatively small area suitable for the treatment of a single or a relatively small number of acne pimples at the same time. The elongated housing 303 has a central axis 371 which extends perpendicularly to the contact surface 327, and the elongated housing 303 is substantially circularly symmetric relative to the central axis 371. The human-tissue treatment device 301 comprises a single light source 305 (e.g., LED) which is arranged on the central axis 371 near a bottom end of the housing 303, i.e., at a relatively large distance from the contact surface 327. The light source 305 is only schematically shown in Fig. 9 and is configured to emit treatment light 321 having an average emission direction perpendicular to the contact surface 327. Alternatively, a small number of light sources (e.g., LEDs) may be arranged near the bottom end of the housing 303 in an annular arrangement closely around the central axis 371.

The human-tissue treatment device 301 further comprises an elongated light guide 373 extending in the elongated housing 303 between the light source 305 and the contact surface 327. The light guide 373 has a central axis which coincides with the central axis 371 of the housing 303 and, accordingly, extends perpendicularly to the contact surface 327. The light guide 373 acts as a collimator 323 of the human-tissue treatment device 301 and has a first end surface 375, which is arranged to receive the treatment light 321 emitted by the light source 305, and a second end surface 377, which is arranged to emit collimated treatment light 321' towards the contact surface 327. In the present embodiment, the light guide 373 comprises a massive light-guiding body 379 made from an optically transparent material, e.g., plastic, sapphire, crystal glass or fused silica. The light-guiding body 379 is separated from the housing 303 by an air gap and may comprise a reflective coating on its circumferential surface 381. The light-guiding body 379 is circularly symmetric relative to the central axis 371 and has a cross-sectional area, perpendicular to the central axis 371, which gradually increases from the first end surface 375 towards the second end surface 377, as shown in Fig. 9. As a result, the light guide 373 is configured to collimate the treatment light 321 received at the first end surface 375 by total internal reflection (TIR) of the received treatment light at the circumferential surface 381 of the light-guiding body 379 and by reflection of the received treatment light at the reflective coating (if present) on the circumferential surface 381.

Alternatively, the light guide may comprise a hollow light-guiding channel (not shown in the figures) configured to collimate the treatment light received at the first end surface by reflection of the received treatment light at a reflective coating provided on an inner surface of the light-guiding channel.

As a result of the circularly symmetric shape of the light-guiding body 379, the gradually increasing cross-sectional area of the light-guiding body 379 in the direction from the first end surface 375 towards the second end surface 377, the arrangement of the light source 305 centrally on the central axis 371, and the average emission direction of the light source 305 perpendicular to the contact surface 327, the light guide 373 provides a relatively high degree of collimation of the collimated treatment light 321' emitted at the second end surface 377. In addition, the degree of collimation and the light intensity of the emitted collimated treatment light 321' are highly uniform over the cross-sectional area of the second end surface 377 of the light guide 373. The light guide 373 constitutes a very practical and structurally simple embodiment of the collimator 323. The first average propagation direction P_{AV1} of the collimated treatment light 321' emitted by the collimator 323 is substantially perpendicularly to the contact surface 327.

As shown in Fig. 9, the human-tissue treatment device 301 further comprises a plate-shaped light output member 325, which comprises the contact surface 327, and a light-redirecting member 353 having a plate-shaped carrier 355, which is arranged immediately below and parallel to the plate-shaped light output member 325. The light-redirecting member 353 is arranged to receive the collimated treatment light 321' emitted by the collimator 323 and having the first average propagation direction P_{AV1}, and to redirect the collimated treatment light 321' from the first average propagation direction P_{AV1} into the second average propagation direction P_{AV2} towards the plate-shaped light output member 325. In essence, the constitution and the optical effects of the plate-shaped light output member 325 and the light-redirecting member 353 are similar to the constitution and the optical effects of the plate-shaped light output member 25 and the light-redirecting member 53 in the third embodiment of the human-tissue treatment device 201 as described in detail hereinbefore. I.e., the plate-shaped light output member 325 has an optical structure 331 similar to the optical structure 31 of the plate-shaped light output member 25, and the light-redirecting member 353 has an optical structure 361 similar to the optical structure 61 of the light-redirecting member 53. The differences are that, whereas the plate-shaped light output member 25 and the light-redirecting member 53 have rectangular circumferential edges, the plate-shaped light output member 325 and the light-redirecting member 353 have circular circumferential edges 383, 385 arranged concentrically relative to the central axis 371, as shown in Fig. 10. Furthermore, whereas the first and second facets 33, 35 of the optical structure 31 of the plate-shaped light output member 25 and the third and fourth facets 63, 65 of the optical structure 61 of the light-redirecting member 53 extend rectilinearly and parallel to each other, the corresponding first and second facets 333, 335 of the corresponding optical structure 331 of the plate-shaped light output member 325 and the corresponding third and fourth facets 363, 365 of the corresponding optical structure 361 of the light-redirecting member 353 are annular and extend concentrically relative to the central axis 371. For simplicity reasons, the optical structures 331 and 361 are not shown in detail in Fig. 9 and will be further explained hereinafter with reference to Fig. 8 and Fig. 10.

In the embodiment shown in Fig. 9, an air gap is present between the second end surface 377 of the light guide 373 and the first main side 357 of the light-redirecting member 353. Alternatively, the first main side 357 of the light-redirecting member 353 may be in direct optical contact with the second end surface 377 of the light guide 373.

In the top view of the human-tissue treatment device 301 in Fig. 10, the annular extension of the first, second, third and fourth facets 333, 335, 363, 365 is indicated schematically by means of only two circular dashed lines C1, C2, while portions of a small number of pairs of first and second facets 333, 335 and the circular edges 337, 339 thereof, and pairs of third and fourth facets 363, 365 and the circular edges 367, 369 thereof are shown in more detail in the detailed top view DTV in Fig. 10. The skilled person will understand that, as a result of the annular extension of the first, second, third and fourth facets 333, 335, 363, 365 about the central axis 371, a cross-section of the plate-shaped light output member 325 and the light-redirecting member 353, taken along any radial line R-R as shown in Fig. 10, will be similar to the cross-section of the plate-shaped light output member 25 and the light-redirecting member 53 of the human-tissue treatment device 201 as schematically shown in Fig. 8. Therefore, for a more detailed description of the geometry and the optical effects of the first, second, third and fourth facets 333, 335, 363, 365 reference is made to the description hereinbefore of the geometry and the optical effects of the corresponding first, second, third and fourth facets 33, 35, 63, 65 of the human-tissue treatment devices 1 and 201. The skilled person will understand that the circular extension of the first, second, third and fourth facets 333, 335, 363, 365 will not basically change the optical effects thereof as compared to the first, second, third and fourth facets 33, 35, 63, 65 of the human-tissue treatment devices 1, 201 as described hereinbefore. In particular, the skilled person will understand that, in the viewing direction of Fig. 10 perpendicular to the contact surface 327, the second average propagation direction P_{AV2} locally extends substantially perpendicularly to the circular edges 337, 339, 367, 369 of the first, second, third and fourth facets 333, 335, 363, 365, i.e., the second average propagation direction P_{AV2} locally extends in a radial plane extending through the central axis 371.

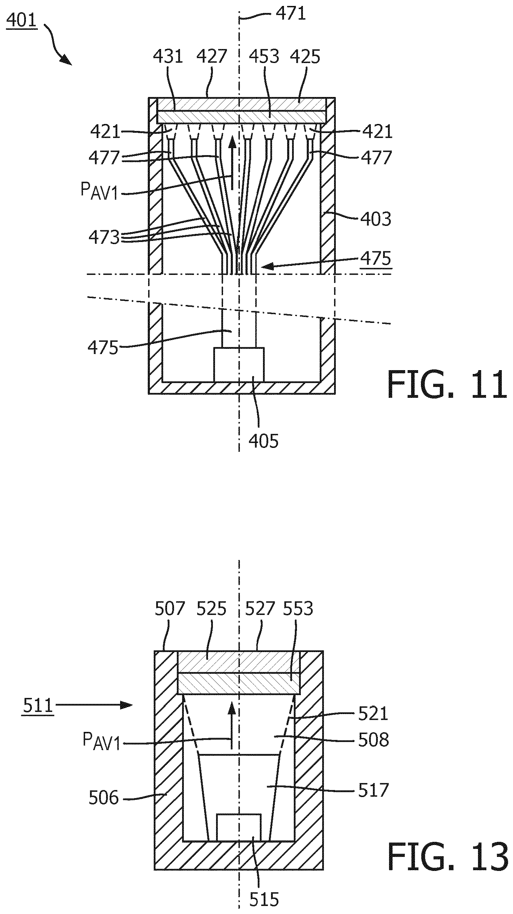

In a fifth embodiment of a human-tissue treatment device 401 according to the invention, a single light source 405 is arranged at a relatively large distance from the plate-shaped light output member 425 comprising the contact surface 427. The treatment light emitted by the light source 405 is guided to the plate-shaped light output member 425 and distributed over the area of the plate-shaped light output member 425 by means of a plurality of optical fibers 473. The human-tissue treatment device 401 is only schematically shown in Fig. 11 and will be discussed only in general terms hereinafter.

The human-tissue treatment device 401 further comprises a light-redirecting member 453. The plate-shaped light output member 425 and the light-redirecting member 453 may be similar to the plate-shaped light output member 325 and the light-redirecting member 353 of the human-tissue treatment device 301 as discussed in detail hereinbefore and, accordingly, may be arranged in a circularly cylindrical housing 403 of the human-tissue treatment device having a central axis 471. Alternatively, the plate-shaped light output member 425 and the light-redirecting member 453 may be similar to the plate-shaped light output member 25 and the light-redirecting member 53 of the human-tissue treatment device 201 as discussed in detail hereinbefore, in which case the housing 403 may have a square or rectangular cross-section perpendicular to the central axis 471. The light source 405 may be arranged at any suitable position within the housing 403, and the optical fibers 473 may be bundled into a bundle 475 extending through the housing 403 from the light source 405 to the light-redirecting member 453. Alternatively, the housing 403 may constitute a treatment handpiece of the human-tissue treatment device 401, and the light source 405 may be arranged in a base station of the human-tissue treatment device 401 which is connected to the treatment handpiece by means of a flexible cable that accommodates the bundle 475 of optical fibers 473.

A person skilled in the art will be able to determine in a straight-forward manner the number and the distribution of the optical fibers 473 required to obtain a desired uniformity of the light intensity of the treatment light at the contact surface 427. In this respect, the number of optical fibers 473 shown in Fig. 11 is purely schematic and illustrative. Furthermore, the optical fibers 473 may act as collimators for the treatment light 421 emitted towards the light-redirecting member 453 by the fiber tip portions 477 of the optical fibers 473. For this purpose, the optical fibers 473 preferably are configured to receive the treatment light emitted by the light source 405 and maintain the received treatment light with a numerical aperture (NA) value in a range from 0.01-0.2. As is generally known to the skilled person, said numerical aperture can be limited to a value within said range by applying a relatively low NA in-coupling of the treatment light into the optical fibers 473 at the position of the light source 405 and/or by using suitable optical properties of the optical fibers 473, in particular of the fiber core material and/or the fiber cladding material. Additional collimating elements, such as ball lenses or micro lenses (not shown in Fig. 11), may be arranged near the fiber tip portions 477 to collimate the treatment light emitted by the fiber tip portions 477, as is also known to the skilled person.

As shown in Fig. 11, the fiber tip portions 477 of the optical fibers 473 are oriented perpendicularly to the contact surface 427 of the plate-shaped light output member 425, so that the first average propagation direction P_{AV1} of the collimated treatment light 421 emitted by the fiber tip portions 477 is perpendicular to the contact surface 427. The light-redirecting member 453 is arranged to redirect the collimated treatment light 421 from the first average propagation direction P_{AV1} into the second average propagation direction P_{AV2} (not shown in Fig. 11) towards the plate-shaped light output member 425 in a manner similar to the light-redirecting member 353 of the human-tissue treatment device 301 as described in detail hereinbefore.

Alternatively, the light-redirecting member 453 of the human-tissue treatment device 401 according to the fifth embodiment may be omitted and the fiber tip portions 477 of the optical fibers 473 may be oriented obliquely relative to the contact surface 427 of the plate-shaped light output member 425, such that the first average propagation direction P_{AV1} of the collimated treatment light 421 emitted by the fiber tip portions 477 is parallel to the second average propagation direction P_{AV2} of the collimated treatment light 421 to be received by the optical structure 431 of the plate-shaped light redirecting member 425. Thus, in this alternative embodiment, the optical fibers 473 are arranged to guide the treatment light emitted by the light source 405 directly to the plate-shaped light output member 425.

The human-tissue treatment devices 1, 101, 201, 301, 401 according to the invention as described hereinbefore are each embodied as a so-called stand-alone device, wherein the treatment of human tissue by means of the collimated treatment light provided at the contact surface 27, 327, 427 is the sole treatment or the main treatment provided by the human-tissue treatment device 1, 101, 201, 301, 401. A human-tissue treatment device according to the invention can also be embodied as a treatment unit of a multifunctional treatment system comprising a main body and a plurality of different treatment units that can be selectively coupled to the main body by the user. An example is a personal care system for the treatment of the human skin, comprising a main body and a plurality of different personal care units that can each be selectively coupled to the main body, wherein the personal care units include, for example, a shaving unit, a hair-trimming unit, a brushing unit, a facial cleansing unit, and a light-therapy unit, embodied as a human-tissue treatment device according to the invention, for the treatment of acne or other skin disorders.

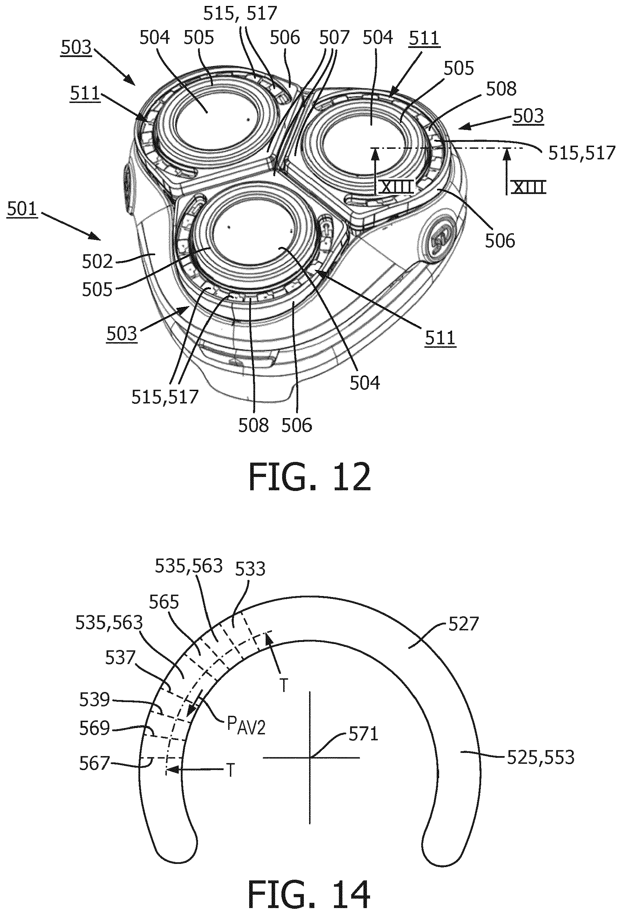

A human-tissue treatment device according to the invention can also be integrated as a secondary treatment unit in a main treatment device, wherein the human-tissue treatment device is arranged in the main treatment device to provide the collimated treatment light to the human tissue together with the main treatment provided by the main treatment device. An example is a shaving unit 501 of an electric shaver according to the invention as shown in Fig. 12. The shaving unit 501 comprises a base member 502 which supports at least one hair-cutting unit 503, in this example three hair-cutting units 503. The hair-cutting units 503 are each of a rotary type and each comprise an external cutting member 504, provided with an annular shaving area 505 with a plurality of hair-entry openings (not shown in detail), and an internal cutting member (not visible) which is covered by the external cutting member 504 and is rotatable within the external cutting member 504. Such a hair-cutting unit is well known to the skilled person and will not be discussed in further detail hereinafter. The shaving unit 501 can be arranged on a main body (not shown) of the electric shaver, which accommodates an electric motor and a transmission system via which the electric motor can drive the internal cutting members of the hair-cutting units 503 into rotation within the external cutting members 504 to cut any hairs that penetrate into the external cutting members 504 via the hair-entry openings in the annular shaving areas 505, as is also well known to the skilled person.

The hair-cutting units 503 of the shaving unit 501 are each at least partially surrounded by a skin-supporting member 506. Each skin-supporting member 506 has a skin-contacting surface 507 which is arranged to be in contact with the skin together with the external cutting members 504 of the hair-cutting units 503 during the shaving process. In the embodiment shown in Fig. 12, each respective skin-supporting member 506 and its skin-contacting surface 507 fully surround the hair-cutting unit 503 associated with the respective skin-supporting member 506. Each respective skin-supporting member 506 comprises a human-tissue treatment device 511 according to the invention, which is arranged in a C-shaped recess 508 provided in the respective skin-supporting member 506 as shown in Fig. 12. In particular, in this example the human-tissue treatment devices 511 are each embodied similar to the third embodiment of the human-tissue treatment device 201 as described hereinbefore, with the main difference that the human-tissue treatment devices 511 each comprise only a single C-shaped row of light sources 515 (e.g., LEDs). Fig. 12 shows how in each respective skin-supporting member 506 the light sources 515 of the associated human-tissue treatment device 511, together with the associated collimating elements 517, are arranged in the C-shaped recess 508 of the respective skin-supporting member 506.

To visualize the light sources 515 in Fig. 12, Fig. 12 does not show the plate-shaped light output members 525 and the light-redirecting members 553 of the human-tissue treatment devices 511. The plate-shaped light output member 525 and the light-redirecting member 553 of one of the human-tissue treatment devices 511 is visible in Fig.13, which schematically shows a cross-section of one of the skin-supporting members 506 extending through the line XIII-XIII in Fig. 12 and perpendicularly to the skin-contacting surface 507. As shown in Fig. 13, in each respective skin-supporting member 506 the contact surface 527 of the plate-shaped light output member 525 of the associated human-tissue treatment device 511 forms a portion of the skin-contacting surface 507 of the respective skin-supporting member 506 and is flush with the skin-supporting surface 507. Although not shown in Fig. 12, it will be evident for the skilled person that the plate-shaped light output members 525 are each C-shaped in order to fully cover the C-shaped recess 508 of the associated skin-supporting member 506.

Two pairs of first and second facets 533, 535 of the plate-shaped light output member 525 and the edges 537, 539 thereof, and two pairs of third and fourth facets 563, 565 of the light-redirecting member 553 and the edges 567, 569 thereof are schematically shown in the top view of one of the human-tissue treatment devices 511 of the shaving unit 501 in Fig. 14. The number of facets and the distance between the facets in Fig. 14 is purely illustrative and schematic. As shown in Fig. 14, the edges 537, 539, 567, 569 of the first, second, third and fourth facets 533, 535, 563, 565 each extend in a radial direction relative to a central axis 571 of the C-shaped light output member 525. Therefore, the skilled person will understand that a cross-section of the plate-shaped light output member 525 and the light-redirecting member 553, taken along the partially circular line T-T as shown in Fig. 14, will be similar to the cross-section of the plate-shaped light output member 25 and the light-redirecting member 53 of the human-tissue treatment device 201 of the third embodiment as schematically shown in Fig. 8. Therefore, for a more detailed description of the geometry and the optical effects of the first, second, third and fourth facets 533, 535, 563, 565 reference is made to the description hereinbefore of the geometry and the optical effects of the corresponding first, second, third and fourth facets 33, 35, 63, 65 of the human-tissue treatment devices 1, 201. The skilled person will understand that the radial extension of the first, second, third and fourth facets 533, 535, 563, 565 will not basically change the optical effects thereof as compared to the first, second, third and fourth facets 33, 35, 63, 65 of the human-tissue treatment devices 1, 201 as described hereinbefore. In particular, the skilled person will understand that, in the viewing direction of Fig. 14 perpendicular to the contact surface 527, the second average propagation direction P_{AV2} locally extends substantially perpendicularly to the edges 537, 539, 567, 569 of the first, second, third and fourth facets 533, 535, 563, 565, as schematically shown in Fig. 14.

During shaving with the shaving unit 501, the contact surfaces 527 of the human-tissue treatment devices 511 arranged in the skin-supporting members 506 of the shaving unit 501 will be in contact with the skin of the user together with the hair-cutting units 503. As a result, the collimated treatment light 521 emitted by the collimating elements 517 of the light sources 515 will be transmitted to the skin via the light-redirected members 553 and the plate-shaped light output members 525 of the human-tissue treatment devices 511 during the shaving process. The collimated treatment light 521 will provide a light therapy to the skin during the shaving process, wherein the type of therapy will depend on the combination of the light intensity and the wavelength of the collimated treatment light 521 as is known to the skilled person.

The scope of the invention is not limited to the embodiments and examples discussed hereinbefore, and several amendments and modifications thereof are possible without deviating from the scope of the invention as defined in the claims. It is intended that the invention be construed as including all such amendments and modifications insofar they come within the scope of the claims. While the invention has been illustrated and described in detail in the figures and the description, such illustration and description are to be considered illustrative or exemplary only, and not restrictive. The drawings are schematic, wherein details which are not required for understanding the invention may have been omitted, and may not necessarily be to scale.

Variations to the disclosed embodiments can be understood and effected by a person skilled in the art in practicing the claimed invention, from a study of the figures, the description and the claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

## Claims

1. A human-tissue treatment device (1) comprising a housing (3), at least one light source (5) arranged in the housing and configured to emit treatment light (15), a collimator (23) configured and arranged to collimate the treatment light emitted by the light source and, thereby, to establish collimated treatment light (21) having a first average propagation direction (P_{AV1}), and a plate-shaped light output member (25) made from an optically transparent material and having a contact surface (27), via which the collimated treatment light is transmissible to a human tissue (41) when the human tissue is in contact with the contact surface, and an inner surface (29) which is opposite to the contact surface and is provided with an optical structure (31), wherein:
- the optical structure (31) of the inner surface (29) of the plate-shaped light output member (25) has one or more pairs of a first facet (33) and a second facet (35), the first facet and the second facet being oriented relative to the contact surface at, respectively, a first angle α1 and a second angle α2;
- the first facets are arranged to receive the collimated treatment light (21), the collimated treatment light having a second average propagation direction (P_{AV2}) at a location where the collimated treatment light is incident on each respective first facet;
- the first angle α1, the second average propagation direction and a refractive index value n1 of the optically transparent material of the plate-shaped light output member are chosen such that: (i) the first facets are configured to transmit the received treatment light (21') into the plate-shaped light output member by refraction; (ii) the contact surface is configured to reflect, at locations of no contact with the human tissue (41), the received treatment light by total internal reflection, TIR; and (iii) the contact surface is configured to transmit, at locations of contact with the human tissue, the received treatment light out of the plate-shaped light output member and into the human tissue by refraction;
- wherein the second facets are arranged to receive the treatment light (21") reflected by TIR at the contact surface; and
- the second angle α2 and the refractive index value n1 are chosen such that the second facets transmit the reflected treatment light (21") out of the plate-shaped light output member by refraction.

2. The human-tissue treatment device (1) as claimed in claim 1, wherein the refractive index value n1 is in a range from 1.4 to 1.7.

3. The human-tissue treatment device (101, 201) as claimed in claim 1 or claim 2, wherein:
- the first average propagation direction (P_{AV1}) is different from the second average propagation direction (P_{AV2}); and
- the treatment device comprises a light-redirecting member (51, 53) arranged between the collimator (23) and the plate-shaped light output member (25) and configured to redirect the collimated treatment light (21) from the first average propagation direction into the second average propagation direction.

4. The human-tissue treatment device (201) as claimed in claim 3, wherein the light-redirecting member (53) comprises a plate-shaped carrier (55) made from an optically transparent material and arranged parallel and adjacent to the inner surface (29) of the plate-shaped light output member (25), and wherein:
- a first main side (57) of the plate-shaped carrier facing away from the plate-shaped light output member is flat, extends parallel to the contact surface (27), and is arranged to receive the collimated treatment light (21) having the first average propagation direction (P_{AV1}) and transmit the received collimated treatment light into the plate-shaped carrier;
- a second main side (59) of the plate-shaped carrier facing the plate-shaped light output member is provided with an optical structure (61) having one or more third facets (63) oriented at a third angle α3 relative to the first main side and arranged to receive the collimated treatment light (221') transmitted by the first main side; and
- the third angle α3, the first average propagation direction and a refractive index value n2 of the optically transparent material of the plate-shaped carrier are chosen such that the third facets are configured to transmit and redirect the received collimated treatment light (221') out of the plate-shaped carrier and into the second average propagation direction (P_{AV2}) by refraction.

5. The human-tissue treatment device (201) as claimed in claim 4, wherein:
- the optical structure (61) of the second main side (59) of the light-redirecting member (53) comprises one or more pairs of a fourth facet (65) and a respective one of the one or more third facets (63), the fourth facet being oriented relative to the first main side (57) of the light-redirecting member at a fourth angle α4; and
- α3 = α2 and α4 = α1.

6. The human-tissue treatment device (301) as claimed in any of the claims 3-5, wherein:
- the collimator (323) comprises an elongated light guide (373) having first and second end surfaces (375, 377) and extending in a direction perpendicular to the contact surface (327) from the first end surface (375) to the second end surface (377);
- the first end surface is arranged to receive the treatment light (321) emitted by the light source (305);
- the second end surface is arranged to transmit the collimated treatment light (321') towards the light-redirecting member (353); and
- the light guide is configured to collimate the treatment light (321) received at the first end surface by internal reflection of the received treatment light at a circumferential surface (381) of the light guide.

7. The human-tissue treatment device (301) as claimed in claim 6, wherein:
- the light guide (373) has a central axis (371) extending perpendicularly to the contact surface (327) and is circularly symmetric relative to the central axis; and
- the light guide has a cross-sectional area perpendicular to the central axis which gradually increases from the first end surface (375) towards the second end surface (377).

8. The human-tissue treatment device (301) as claimed in claim 6 or claim 7, wherein the light guide (373) comprises a massive light-guiding body (379) made from an optically transparent material and configured to collimate the treatment light (321) received at the first end surface (375) by TIR of the received treatment light at a circumferential surface (381) of the light-guiding body.

9. The human-tissue treatment device (301) as claimed in claim 7, wherein the first and second facets (333, 335) of the optical structure (331) of the plate-shaped light output member (325) are annular and extend concentrically relative to the central axis (371) of the light guide (373).

10. The human-tissue treatment device (201) as claimed in claim 4 or claim 5, comprising a plurality of light sources (5) configured to emit the treatment light, wherein the collimator (23) comprises a separate collimating element (7) for each respective light source configured and arranged to collimate the treatment light emitted by the respective light source.

11. The human-tissue treatment device (101) as claimed in claim 3, the at least one light source (5) comprising a plurality of light sources (5) configured to emit the treatment light, wherein:
- the collimator (23) comprises a separate collimating element (7) for each respective light source configured and arranged to collimate the treatment light emitted by the respective light source; and
- the light-redirecting member (51) comprises a separate light-redirecting element (43) for each respective light source arranged between the plate-shaped light output member (25) and the collimating element associated with the respective light source.

12. The human-tissue treatment device (101) as claimed in claim 11, wherein each light-redirecting element (43) comprises a mirror (47) or a prism (49).

13. The human-tissue treatment device (1) as claimed in claim 1 or claim 2, the at least one light source (5) comprising a plurality of light sources (5) configured to emit the treatment light, wherein:
- the collimator (23) comprises a separate collimating element (7) for each respective light source configured and arranged to collimate the treatment light emitted by the respective light source; and
- each respective light source and each respective collimating element associated with the respective light source are oriented obliquely relative to the contact surface (27) of the plate-shaped light output member (25), such that the first average propagation direction (P_{AV1}) and the second average propagation direction (P_{AV2}) are mutually parallel.

14. The human-tissue treatment device as claimed in claim 1 or claim 2, wherein:
- the collimator comprises a plurality of optical fibers arranged to guide the treatment light emitted by the light source to the plate-shaped light output member;
- the optical fibers have fiber tip portions which are oriented such that the treatment light emitted by the fiber tip portions has the second average propagation direction; and
- the optical fibers are configured to receive the treatment light emitted by the light source and maintain the received treatment light with a numerical aperture value in a range of 0.01-0.2.

15. The human-tissue treatment device (401) as claimed in any of the claims 3-5, wherein:
- the collimator comprises a plurality of optical fibers (473) arranged to guide the treatment light emitted by the light source (405) to the light-redirecting member (453);
- the optical fibers have fiber tip portions (477) which are oriented perpendicularly to the contact surface (427) of the plate-shaped light output member (425); and
- the optical fibers are configured to receive the treatment light emitted by the light source and maintain the received treatment light with a numerical aperture value in a range of 0.01-0.2.

16. A shaving unit (501) of an electric shaver, comprising a base member (502), at least one hair-cutting unit (503) supported by the base member, and at least one human-tissue treatment device (511) as claimed in any of the claims 1-15, wherein the contact surface (527) of the plate-shaped light output member (525) of the human-tissue treatment device is arranged relative to the hair-cutting unit such that the contact surface and the hair-cutting unit are together in contact with skin of a user during use of the shaving unit.

17. The shaving unit (501) as claimed in claim 16, further comprising a skin-supporting member (506) having a skin-supporting surface (507) which at least partially surrounds the hair-cutting unit (503), wherein the human-tissue treatment device (511) is arranged in the skin-supporting member such that the contact surface (527) of the plate-shaped light output member (525) of the human-tissue treatment device (511) forms a portion of the skin-contacting surface of the skin-supporting member.

18. An electric shaver comprising a main body and a shaving unit (501) as claimed in claim 16 or 17, wherein the main body accommodates an electric motor, and wherein the shaving unit is arranged on the main body such that the hair-cutting unit (503) of the shaving unit is drivable by the electric motor.

## Patentansprüche

1. Vorrichtung (1) zur Behandlung von menschlichem Gewebe, umfassend ein Gehäuse (3), mindestens eine Lichtquelle (5), die im Gehäuse angeordnet ist und so konfiguriert ist, dass sie Behandlungslicht (15) emittiert, einen Kollimator (23), der so konfiguriert und angeordnet ist, dass er das von der Lichtquelle emittierte Behandlungslicht kollimiert und dadurch kollimiertes Behandlungslicht (21) mit einer ersten mittleren Ausbreitungsrichtung (P_{AV1}) erzeugt, und ein plattenförmiges Lichtausgabeelement (25), das aus einem optisch transparenten Material gefertigt ist und eine Kontaktfläche (27), über die das kollimierte Behandlungslicht auf ein menschliches Gewebe (41) durchgelassen wird, wenn das menschliche Gewebe mit der Kontaktfläche in Kontakt steht, und eine Innenfläche (29) aufweist, die der Kontaktfläche gegenüberliegt und mit einer optischen Struktur (31) versehen ist, wobei;
- die optische Struktur (31) der Innenfläche (29) des plattenförmigen Lichtausgabeelements (25) ein oder mehrere Paare einer ersten Facette (33) und einer zweiten Facette (35) aufweist, wobei die erste Facette und die zweite Facette relativ zur Kontaktfläche in einem ersten Winkel α1 bzw. einem zweiten Winkel α2 orientiert sind;
- die ersten Facetten so angeordnet sind, dass sie das kollimierte Behandlungslicht (21) aufnehmen, wobei das kollimierte Behandlungslicht eine zweite mittlere Ausbreitungsrichtung (P_{AV2}) an einer Stelle aufweist, an der das kollimierte Behandlungslicht auf die jeweilige erste Facette trifft;
- der erste Winkel α1, die zweite mittlere Ausbreitungsrichtung und ein Brechungsindexwert n1 des optisch transparenten Materials des plattenförmigen Lichtausgabeelements so gewählt sind, dass: (i) die ersten Facetten so konfiguriert sind, dass sie das empfangene Behandlungslicht (21') durch Brechung in das plattenförmige Lichtausgabeelement durchlassen; ii) die Kontaktfläche so konfiguriert ist, dass sie an Stellen ohne Kontakt zum menschlichen Gewebe (41) das empfangene Behandlungslicht durch Totalreflexion, TIR, reflektiert; und (iii) die Kontaktfläche so konfiguriert ist, dass sie an Stellen mit Kontakt zum menschlichen Gewebe das empfangene Behandlungslicht durch Brechung aus dem plattenförmigen Lichtausgabeelement und in das menschliche Gewebe überträgt;
- wobei die zweiten Facetten so angeordnet sind, dass sie das durch TIR an der Kontaktfläche reflektierte Behandlungslicht (21") aufnehmen; und
- der zweite Winkel α2 und der Brechungsindex n1 so gewählt sind, dass die zweiten Facetten das reflektierte Behandlungslicht (21") durch Brechung aus dem plattenförmigen Lichtausgabeelement durchlassen.

2. Vorrichtung (1) zur Behandlung von menschlichem Gewebe nach Anspruch 1, wobei der Brechungsindexwert n1 im Bereich von 1,4 bis 1,7 liegt.

3. Vorrichtung (101, 201) zur Behandlung von menschlichem Gewebe nach Anspruch 1 oder Anspruch 2, wobei:
- die erste mittlere Ausbreitungsrichtung (P_{AV1}) sich von der zweiten mittleren Ausbreitungsrichtung (P_{AV2}) unterscheidet; und
- die Behandlungsvorrichtung ein Lichtumlenkelement (51, 53) umfasst, das zwischen dem Kollimator (23) und dem plattenförmigen Lichtausgabeelement (25) angeordnet ist und so konfiguriert ist, dass es das kollimierte Behandlungslicht (21) von der ersten mittleren Ausbreitungsrichtung in die zweite mittlere Ausbreitungsrichtung umlenkt.

4. Vorrichtung (201) zur Behandlung von menschlichem Gewebe nach Anspruch 3, wobei das Lichtumlenkelement (53) einen plattenförmigen Träger (55) umfasst, der aus einem optisch transparenten Material gefertigt ist und parallel und angrenzend an die Innenfläche (29) des plattenförmigen Lichtauslasselements (25) angeordnet ist, und wobei:
- eine erste Hauptseite (57) des plattenförmigen Trägers, die vom plattenförmigen Lichtausgabeelement abgewandt ist, flach ist, sich parallel zur Kontaktfläche (27) erstreckt und so angeordnet ist, dass sie das kollimierte Behandlungslicht (21) mit der ersten mittleren Ausbreitungsrichtung (P_{AV1}) aufnimmt und das empfangene kollimierte Behandlungslicht in den plattenförmigen Träger durchlässt;
- eine zweite Hauptseite (59) des plattenförmigen Trägers, die dem plattenförmigen Lichtausgabeelement zugewandt ist, mit einer optischen Struktur (61) versehen ist, die eine oder mehrere dritte Facetten (63) aufweist, die in einem dritten Winkel α3 relativ zur ersten Hauptseite ausgerichtet sind und so angeordnet sind, dass sie das von der ersten Hauptseite durchgelassene kollimierte Behandlungslicht (221') empfangen; und
- der dritte Winkel α3, die erste mittlere Ausbreitungsrichtung und ein Brechungsindexwert n2 des optisch transparenten Materials des plattenförmigen Trägers so gewählt sind, dass die dritten Facetten so konfiguriert sind, dass sie das empfangene kollimierte Behandlungslicht (221') aus dem plattenförmigen Träger heraus durchlassen und durch Brechung in die zweite mittlere Ausbreitungsrichtung (P_{AV2}) umlenken.

5. Vorrichtung (201) zur Behandlung von menschlichem Gewebe nach Anspruch 4, wobei:
- die optische Struktur (61) der zweiten Hauptseite (59) des Lichtumlenkelements (53) ein oder mehrere Paare einer vierten Facette (65) und jeweils einer oder mehrerer dritter Facetten (63) umfasst, wobei die vierte Facette relativ zur ersten Hauptseite (57) des Lichtumlenkelements in einem vierten Winkel α4 orientiert ist; und
- α3 = α2 und α4 = α1.

6. Vorrichtung (301) zur Behandlung von menschlichem Gewebe nach einem der Ansprüche 3-5, wobei:
- der Kollimator (323) einen länglichen Lichtleiter (373) umfasst, der eine erste und eine zweite Stirnfläche (375, 377) aufweist und sich in einer Richtung senkrecht zur Kontaktfläche (327) von der ersten Stirnfläche (375) zur zweiten Stirnfläche (377) erstreckt;
- die erste Stirnfläche so angeordnet ist, dass sie das von der Lichtquelle (305) emittierte Behandlungslicht (321) empfängt;
- die zweite Stirnfläche so angeordnet ist, dass sie das kollimierte Behandlungslicht (321') zu dem Lichtumlenkelement (353) durchlässt; und
- der Lichtleiter so konfiguriert ist, dass er das an der ersten Stirnfläche empfangene Behandlungslicht (321) durch interne Reflexion des empfangenen Behandlungslichts an einer Umfangsfläche (381) des Lichtleiters kollimiert.

7. Vorrichtung (301) zur Behandlung von menschlichem Gewebe nach Anspruch 6, wobei:
- der Lichtleiter (373) eine Mittelachse (371) aufweist, die sich senkrecht zur Kontaktfläche (327) erstreckt und relativ zu der Mittelachse kreissymmetrisch ist; und
- der Lichtleiter eine Querschnittsfläche senkrecht zur Mittelachse aufweist, die von der ersten Stirnfläche (375) zur zweiten Stirnfläche (377) hin allmählich zunimmt.

8. Vorrichtung (301) zur Behandlung von menschlichem Gewebe nach Anspruch 6 oder Anspruch 7, wobei der Lichtleiter (373) einen massiven Lichtleiterkörper (379) umfasst, der aus einem optisch transparenten Material gefertigt und so konfiguriert ist, dass er das an der ersten Stirnfläche (375) empfangene Behandlungslicht (321) durch TIR des empfangenen Behandlungslichts an einer Umfangsfläche (381) des Lichtleiterkörpers kollimiert.

9. Vorrichtung (301) zur Behandlung von menschlichem Gewebe nach Anspruch 7, wobei die ersten und zweiten Facetten (333, 335) der optischen Struktur (331) des plattenförmigen Lichtausgabeelements (325) ringförmig sind und sich konzentrisch zur Mittelachse (371) des Lichtleiters (373) erstrecken.

10. Vorrichtung (201) zur Behandlung von menschlichem Gewebe nach Anspruch 4 oder Anspruch 5, umfassend eine Vielzahl von Lichtquellen (5), die so konfiguriert sind, dass sie das Behandlungslicht emittieren, wobei der Kollimator (23) für jede einzelne Lichtquelle ein separates Kollimatorelement (7) umfasst, das so konfiguriert und angeordnet ist, dass es das von der jeweiligen Lichtquelle emittierte Behandlungslicht kollimiert.

11. Vorrichtung (101) zur Behandlung von menschlichem Gewebe nach Anspruch 3, wobei die mindestens eine Lichtquelle (5) eine Vielzahl von Lichtquellen (5) umfasst, die so konfiguriert sind, dass sie das Behandlungslicht emittieren, wobei:
- der Kollimator (23) für jede Lichtquelle ein separates Kollimatorelement (7) umfasst, das so konfiguriert und angeordnet ist, dass es das von der jeweiligen Lichtquelle emittierte Behandlungslicht kollimiert; und
- das Lichtumlenkelement (51) für jede Lichtquelle ein separates Lichtumlenkelement (43) umfasst, das zwischen dem plattenförmigen Lichtauslasselement (25) und dem der jeweiligen Lichtquelle zugeordneten Kollimatorelement angeordnet ist.

12. Vorrichtung (101) zur Behandlung von menschlichem Gewebe nach Anspruch 11, wobei jedes Lichtumlenkelement (43) einen Spiegel (47) oder ein Prisma (49) umfasst.

13. Vorrichtung (1) zur Behandlung von menschlichem Gewebe nach Anspruch 1 oder Anspruch 2, wobei die mindestens eine Lichtquelle (5) eine Vielzahl von Lichtquellen (5) umfasst, die so konfiguriert sind, dass sie das Behandlungslicht emittieren, wobei:
- der Kollimator (23) für jede Lichtquelle ein separates Kollimatorelement (7) umfasst, das so konfiguriert und angeordnet ist, dass es das von der jeweiligen Lichtquelle emittierte Behandlungslicht kollimiert; und
- jede jeweilige Lichtquelle und jedes jeweilige Kollimatorelement, das der jeweiligen Lichtquelle zugeordnet ist, schräg zur Kontaktfläche (27) des plattenförmigen Lichtausgabeelements (25) ausgerichtet sind, sodass die erste mittlere Ausbreitungsrichtung (P_{AV1}) und die zweite mittlere Ausbreitungsrichtung (P_{AV2}) zueinander parallel sind.

14. Vorrichtung zur Behandlung von menschlichem Gewebe nach Anspruch 1 oder Anspruch 2, wobei:
- der Kollimator eine Vielzahl von optischen Fasern umfasst, die so angeordnet sind, dass sie das von der Lichtquelle emittierte Behandlungslicht zum plattenförmigen Lichtausgabeelement leiten;
- die optischen Fasern Faserspitzenabschnitte aufweisen, die so ausgerichtet sind, dass das von den Faserspitzenabschnitten emittierte Behandlungslicht die zweite mittlere Ausbreitungsrichtung aufweist; und
- die optischen Fasern so konfiguriert sind, dass sie das von der Lichtquelle emittierte Behandlungslicht empfangen und das empfangene Behandlungslicht mit einem numerischen Aperturwert im Bereich von 0,01-0,2 aufrechterhalten.

15. Vorrichtung (401) zur Behandlung von menschlichem Gewebe nach einem der Ansprüche 3-5, wobei:
- der Kollimator eine Vielzahl von optischen Fasern (473) umfasst, die so angeordnet sind, dass sie das von der Lichtquelle (405) emittierte Behandlungslicht zum Lichtumlenkelement (453) leiten;
- die optischen Fasern Faserspitzenabschnitte (477) aufweisen, die senkrecht zur Kontaktfläche (427) des plattenförmigen Lichtausgabeelements (425) ausgerichtet sind; und
- die optischen Fasern so konfiguriert sind, dass sie das von der Lichtquelle emittierte Behandlungslicht empfangen und das empfangene Behandlungslicht mit einem numerischen Aperturwert im Bereich von 0,01-0,2 aufrechterhalten.

16. Rasiereinheit (501) eines Elektrorasierers, umfassend ein Basiselement (502), mindestens eine Haarschneideeinheit (503), die vom Basiselement getragen wird, und mindestens eine Vorrichtung (511) zur Behandlung von menschlichem Gewebe nach einem der Ansprüche 1-15, wobei die Kontaktfläche (527) des plattenförmigen Lichtausgabeelements (525) der Vorrichtung zur Behandlung von menschlichem Gewebe relativ zur Haarschneideeinheit so angeordnet ist, dass die Kontaktfläche und die Haarschneideeinheit während der Verwendung der Rasiereinheit gemeinsam mit der Haut eines Benutzers in Kontakt stehen.

17. Rasiereinheit (501) nach Anspruch 16, ferner umfassend ein Hautstützelement (506) mit einer Hautstützfläche (507), die die Haarschneideeinheit (503) mindestens teilweise umschließt, wobei die Vorrichtung (511) zur Behandlung von menschlichem Gewebe in dem Hautstützelement so angeordnet ist, dass die Kontaktfläche (527) des plattenförmigen Lichtausgabeelements (525) der Vorrichtung (511) zur Behandlung von menschlichem Gewebe einen Abschnitt der Hautkontaktfläche des Hautstützelements bildet.

18. Elektrischer Rasierer, umfassend einen Hauptkörper und eine Rasiereinheit (501) nach Anspruch 16 oder 17, wobei der Hauptkörper einen Elektromotor aufnimmt und wobei die Rasiereinheit so auf dem Hauptkörper angeordnet ist, dass die Haarschneideeinheit (503) der Rasiereinheit vom Elektromotor angetrieben werden kann.

## Revendications

1. Dispositif de traitement de tissu humain (1) comprenant un boîtier (3), au moins une source de lumière (5) agencée dans le boîtier et configurée pour émettre une lumière de traitement (15), un collimateur (23) configuré et agencé pour collimater la lumière de traitement émise par la source de lumière et, par conséquent, pour établir une lumière de traitement collimatée (21) présentant une première direction de propagation moyenne (P_{AV1}), et un élément de sortie de lumière en forme de plaque (25) fabriqué à partir d'un matériau optiquement transparent et présentant une surface de contact (27), par l'intermédiaire de laquelle la lumière de traitement collimatée est transmissible à un tissu humain (41) lorsque le tissu humain est en contact avec la surface de contact, et une surface interne (29) opposée à la surface de contact et dotée d'une structure optique (31), dans lequel ;
- la structure optique (31) de la surface interne (29) de l'élément de sortie de lumière en forme de plaque (25) présente une ou plusieurs paires d'une première facette (33) et d'une deuxième facette (35), la première facette et la deuxième facette étant orientées par rapport à la surface de contact selon un premier angle α1 et un deuxième angle α2 ;
- les premières facettes sont agencées pour recevoir la lumière de traitement collimatée (21), la lumière de traitement collimatée présentant une seconde direction de propagation moyenne (P_{AV2}) à un endroit où la lumière de traitement collimatée est incidente sur chaque première facette respective ;
- le premier angle α1, la seconde direction de propagation moyenne et une valeur d'indice de réfraction n1 du matériau optiquement transparent de l'élément de sortie de lumière en forme de plaque sont choisis de telle sorte que : (i) les premières facettes soient configurées pour transmettre la lumière de traitement reçue (21') dans l'élément de sortie de lumière en forme de plaque par réfraction ; ii) la surface de contact soit configurée pour réfléchir, aux endroits sans contact avec le tissu humain (41), la lumière de traitement reçue par réflexion totale interne, TIR ; et (iii) la surface de contact soit configurée pour transmettre, aux endroits de contact avec le tissu humain, la lumière de traitement reçue hors de l'élément de sortie de lumière en forme de plaque et dans le tissu humain par réfraction ;
- dans lequel les deuxièmes facettes sont agencées pour recevoir la lumière de traitement (21") réfléchie par TIR au niveau de la surface de contact ; et
- le deuxième angle α2 et la valeur de l'indice de réfraction n1 sont choisis de telle sorte que les deuxièmes facettes transmettent la lumière de traitement (21") réfléchie hors de l'élément de sortie de lumière en forme de plaque par réfraction.

2. Dispositif de traitement de tissu humain (1) selon la revendication 1, dans lequel la valeur d'indice de réfraction n1 est dans une plage de 1,4 à 1,7.

3. Dispositif de traitement de tissu humain (101, 201) selon la revendication 1 ou la revendication 2, dans lequel :
- la première direction de propagation moyenne (P_{AV1}) est différente de la seconde direction de propagation moyenne (P_{AV2)}) ; et
- le dispositif de traitement comprend un élément de redirection de la lumière (51, 53) agencé entre le collimateur (23) et l'élément de sortie de lumière en forme de plaque (25) et configuré pour rediriger la lumière de traitement collimatée (21) de la première direction de propagation moyenne dans la seconde direction de propagation moyenne.

4. Dispositif de traitement de tissu humain (201) selon la revendication 3, dans lequel l'élément de redirection de la lumière (53) comprend un support en forme de plaque (55) fabriqué à partir d'un matériau optiquement transparent et agencé parallèlement et de manière adjacente à la surface interne (29) de l'élément de sortie de lumière en forme de plaque (25), et dans lequel :
- un premier côté principal (57) du support en forme de plaque orienté à l'opposé de l'élément de sortie de lumière en forme de plaque est plat, s'étend parallèlement à la surface de contact (27) et est agencé pour recevoir la lumière de traitement collimatée (21) présentant la première direction de propagation moyenne (P_{AV1}) et transmettre la lumière de traitement collimatée reçue dans le support en forme de plaque ;
- un second côté principal (59) du support en forme de plaque faisant face à l'élément de sortie de lumière en forme de plaque est doté d'une structure optique (61) présentant une ou plusieurs troisièmes facettes (63) orientées selon un troisième angle α3 par rapport au premier côté principal et agencées pour recevoir la lumière de traitement collimatée (221') transmise par le premier côté principal ; et
- le troisième angle α3, la première direction de propagation moyenne et une valeur d'indice de réfraction n2 du matériau optiquement transparent du support en forme de plaque sont choisis de telle sorte que les troisièmes facettes soient configurées pour transmettre et rediriger la lumière de traitement collimatée (221') reçue hors du support en forme de plaque et dans la seconde direction de propagation moyenne (P_{AV2}) par réfraction.

5. Dispositif de traitement de tissu humain (201) selon la revendication 4, dans lequel :
- la structure optique (61) du deuxième côté principal (59) de l'élément de redirection de la lumière (53) comprend une ou plusieurs paires d'une quatrième facette (65) et d'une respective des une ou plusieurs troisièmes facettes (63), la quatrième facette étant orientée par rapport au premier côté principal (57) de l'élément de redirection de la lumière selon un quatrième angle α4 ; et
- α3 = α2 et α4 = α1.

6. Dispositif de traitement de tissu humain (301) selon l'une quelconque des revendications 3-5, dans lequel :
- le collimateur (323) comprend un guide de lumière allongé (373) présentant des première et seconde surfaces d'extrémité (375, 377) et s'étendant dans une direction perpendiculaire à la surface de contact (327) de la première surface d'extrémité (375) à la seconde surface d'extrémité (377) ;
- la première surface d'extrémité est agencée pour recevoir la lumière de traitement (321) émise par la source de lumière (305) ;
- la seconde surface d'extrémité est agencée pour transmettre la lumière de traitement collimatée (321') vers l'élément de redirection de la lumière (353) ; et
- le guide de lumière est configuré pour collimater la lumière de traitement (321) reçue au niveau de la première surface d'extrémité par réflexion interne de la lumière de traitement reçue au niveau d'une surface circonférentielle (381) du guide de lumière.

7. Dispositif de traitement de tissu humain (301) selon la revendication 6, dans lequel :
- le guide de lumière (373) présente un axe central (371) s'étendant perpendiculairement à la surface de contact (327) et présente une symétrie de révolution par rapport à l'axe central ; et
- le guide de lumière présente une section transversale perpendiculaire à l'axe central qui augmente progressivement de la première surface d'extrémité (375) vers la seconde surface d'extrémité (377).

8. Dispositif de traitement de tissu humain (301) selon la revendication 6 ou la revendication 7, dans lequel le guide de lumière (373) comprend un corps de guidage de lumière massif (379) fabriqué à partir d'un matériau optiquement transparent et configuré pour collimater la lumière de traitement (321) reçue au niveau de la première surface d'extrémité (375) par TIR de la lumière de traitement reçue au niveau d'une surface circonférentielle (381) du corps de guidage de lumière.

9. Dispositif de traitement de tissu humain (301) selon la revendication 7, dans lequel les première et deuxième facettes (333, 335) de la structure optique (331) de l'élément de sortie de lumière en forme de plaque (325) sont annulaires et s'étendent de manière concentrique par rapport à l'axe central (371) du guide de lumière (373).

10. Dispositif de traitement de tissu humain (201) selon la revendication 4 ou la revendication 5, comprenant une pluralité de sources de lumière (5) configurées pour émettre la lumière de traitement, dans lequel le collimateur (23) comprend un élément de collimation distinct (7) pour chaque source de lumière respective configurée et agencée pour collimater la lumière de traitement émise par la source de lumière respective.

11. Dispositif de traitement de tissu humain (101) selon la revendication 3, l'au moins une source de lumière (5) comprenant une pluralité de sources de lumière (5) configurées pour émettre la lumière de traitement, dans lequel :
- le collimateur (23) comprend un élément de collimation distinct (7) pour chaque source de lumière respective, configuré et agencé pour collimater la lumière de traitement émise par la source de lumière respective ; et
- l'élément de redirection de la lumière (51) comprend un élément de redirection de la lumière distinct (43) pour chaque source de lumière respective agencé entre l'élément de sortie de lumière en forme de plaque (25) et l'élément de collimation associé à la source de lumière respective.

12. Dispositif de traitement de tissu humain (101) selon la revendication 11, dans lequel chaque élément de redirection de la lumière (43) comprend un miroir (47) ou un prisme (49).

13. Dispositif de traitement de tissu humain (1) selon la revendication 1 ou la revendication 2, l'au moins une source de lumière (5) comprenant une pluralité de sources de lumière (5) configurées pour émettre la lumière de traitement, dans lequel :
- le collimateur (23) comprend un élément de collimation distinct (7) pour chaque source de lumière respective, configuré et agencé pour collimater la lumière de traitement émise par la source de lumière respective ; et
- chaque source de lumière respective et chaque élément de collimation respectif associé à la source de lumière respective sont orientés obliquement par rapport à la surface de contact (27) de l'élément de sortie de lumière en forme de plaque (25), de telle sorte que la première direction de propagation moyenne (P_{AV1}) et la seconde direction de propagation moyenne (P_{AV2}) soient mutuellement parallèles.

14. Dispositif de traitement de tissu humain selon la revendication 1 ou la revendication 2, dans lequel :
- le collimateur comprend une pluralité de fibres optiques agencées pour guider la lumière de traitement émise par la source de lumière vers l'élément de sortie de lumière en forme de plaque ;
- les fibres optiques présentent des parties d'extrémité de fibre qui sont orientées de telle sorte que la lumière de traitement émise par ces parties d'extrémité présente la seconde direction de propagation moyenne ; et
- les fibres optiques sont configurées pour recevoir la lumière de traitement émise par la source de lumière et maintenir la lumière de traitement reçue avec une valeur d'ouverture numérique dans une plage de 0,01 à 0,2.

15. Dispositif de traitement de tissu humain (401) selon l'une quelconque des revendications 3-5, dans lequel :
- le collimateur comprend une pluralité de fibres optiques (473) agencées pour guider la lumière de traitement émise par la source de lumière (405) vers l'élément de redirection de la lumière (453) ;
- les fibres optiques présentent des parties d'extrémité de fibre (477) qui sont orientées perpendiculairement à la surface de contact (427) de l'élément de sortie de lumière en forme de plaque (425) ; et
- les fibres optiques sont configurées pour recevoir la lumière de traitement émise par la source de lumière et maintenir la lumière de traitement reçue avec une valeur d'ouverture numérique dans une plage de 0,01 à 0,2.

16. Unité de rasage (501) d'un rasoir électrique, comprenant un élément de base (502), au moins une unité de coupe de poils (503) supportée par l'élément de base et au moins un dispositif de traitement de tissu humain (511) selon l'une quelconque des revendications 1-15, dans laquelle la surface de contact (527) de l'élément de sortie de lumière en forme de plaque (525) du dispositif de traitement de tissu humain est agencée par rapport à l'unité de coupe de poils de telle sorte que la surface de contact et le dispositif de coupe de poils soient ensemble en contact avec la peau d'un utilisateur pendant l'utilisation de l'unité de rasage.

17. Unité de rasage (501) selon la revendication 16, comprenant en outre un élément d'appui sur la peau (506) présentant une surface d'appui sur la peau (507) qui entoure au moins partiellement l'unité de coupe de poils (503), dans laquelle le dispositif de traitement de tissu humain (511) est agencé dans l'élément d'appui sur la peau de telle sorte que la surface de contact (527) de l'élément de sortie de lumière en forme de plaque (525) du dispositif de traitement de tissu humain (511) forme une partie de la surface de contact avec la peau de l'élément d'appui sur la peau.

18. Rasoir électrique comprenant un corps principal et une unité de rasage (501) selon la revendication 16 ou 17, dans lequel le corps principal abrite un moteur électrique, et dans lequel l'unité de rasage est agencée sur le corps principal de telle sorte que l'unité de coupe de poils (503) de l'unité de rasage soit entraînée par le moteur électrique.
